**Europäisches Patentamt**

**European Patent Office**

**Office européen des brevets**

(11) Veröffentlichungsnummer: **0 392 424**
**A1**

## (12) EUROPÄISCHE PATENTANMELDUNG

(21) Anmeldenummer: **90106770.2**

(22) Anmeldetag: **09.04.90**

(51) Int. Cl.⁵: **A61C 5/00**

(30) Priorität: **08.04.89 DE 3911551**

(43) Veröffentlichungstag der Anmeldung:
**17.10.90 Patentblatt 90/42**

(84) Benannte Vertragsstaaten:
**AT BE CH DE DK ES FR GB GR IT LI LU NL SE**

(71) Anmelder: **Arndt, Helge**
**Grosse Bergstrasse 40 A**
**D-3007 Gehrden 1(DE)**

(72) Erfinder: **Arndt, Helge**
**Grosse Bergstrasse 40 A**
**D-3007 Gehrden 1(DE)**

(54) Gerät zum Aufbringen eines metallischen Stoffes auf Zähnen und auf Zähnen befindlichen körperfremden Materialien.

㊷ Die Erfindung betrifft ein Gerät zum Aufbringen eines metallischen Stoffes auf Zähnen und auf den Zähnen befindlichen körperfremden Materialien.

Der Erfindung liegt die Aufgabe zugrunde, ein einfach herstellbares, gut handhabbares zahnärztliches Werkzeug zu schaffen, mit dem sich im Mund des Patienten Metall auf Zähnen oder auf den Zähnen befindlichen körperfremden Materialien festhaftend, in kürzester Zeit, mit minimaler Gefahr für den Patienten, auch an schwer zugänglichen Stellen und in einfacher Weise aufbringen läßt.

Die Erfindung besteht bei dem eingangs genannten Gerät aus

einer an einen Behälter für eine Metall enthaltende Flüssigkeit angeschlossene, Auftragvorrichtung und

einer weiteren, mit dieser zu einer Einheit zusammengebauten Auftragvorrichtung für das Mittel zum Abscheiden des Metalls, vorzugsweise eines Reduktionsmittels,

und Vorrichtungen für die gleichzeitige oder in kleinen Zeitintervallen alternierend erfolgende Zuführung zu den Auftragvorrichtungen,

wobei die Auftragvorrichtungen mit ihren Ausgängen auf einen gemeinsamen Ort oder zwei dicht nebeneinander liegenden Orten gerichtet sind.

EP 0 392 424 A1

## Gerät zum Aufbringen eines metallischen Stoffes auf Zähnen und auf den Zähnen befindlichen körperfremden Materialien

Die Erfindung betrifft ein Gerät nach dem Oberbegriff des Anspruchs 1.

In einem neuartigen zahnärztlichen therapeutischen Verfahren wird ein zu behandelnder Zahn nach grundsätzlich konventioneller Präparation und spezieller Vorbehandlung, je nach Füllungsart und -randlage bis, vorzugsweise geringfügig über die Präparationsgrenze hinaus, mit einem substanzundurchlässigen metallischen Belag versehen und die Füllung oder Krone auf diesen appliziert. Nachträglich wird in einigen Fällen auch der Füllungsrand, insbesondere die Zementfuge zum Schutz vor Zementauswaschung mit Metall belegt. Die über die Präparationsgrenze hinausgehenden zu beschichtenden Flächen stellen dabei im wesentlichen die in vivo nicht oder nur schwer erreichbaren Stellen (Approximalflächen/ subgingivale Flächen), die einer besonders hochqualitativen, haftfesten und gleichmäßigen Metallaufbringung bedürfen dar. Weiterhin schließt das Verfahren die Metallauftragung auf die Wurzelkanalwandung eines aufbereiteten Kanals ein. In der Medizin wird eine Metallauftragung auf Knochen, beispielsweise zur Erhöhung der Haftfestigkeit einer Endoprothese, durchgeführt.

In den französischen und amerikanischen Patentschriften FR-OS 2 287 208 (09.10.75) und US-PS 3 995 371 (10.10.74) ist ein Verfahren zur Behandlung von Zähnen und Knochen durch stromlose Metallbeschichtung bekannt geworden, mit dem sich Amalgam- und Goldhämmerfüllungen haftfest applizieren und Fissuren oder Grübchen versiegeln lassen.

Weitere Veröffentlichungen: (DIMDINET)

1.: Cutright D.E.; Woody R.D.; O'Keefe T.J.; Hoffman J.I.; "The use of electroless metallcoatings as preventive materials - a preliminary primate study" J Prev Dent 4:37-42, 1977

2.: O'Keefe T.J.; Christie R.D.; "Electroless and chemical metaldepositing on human tooth." J.Dent Research Vol. 56,1977.

3.: O'Keefe T.J.; Narasagoudar R.A.; Anderson L.A.; "Depositing of rate of metallic silver on ivory and enamel." J.Dent Research Vol. 61, No.2 1982/Feb.

Bei diesem Metallauftragungsverfahren werden die Metall und Reduktionsmittel enthaltenden Elektrolytlösungen mittels eines Wattebausches, beispielsweise einem Q-Tip, oder im Tauchverfahren aufgebracht. Die Abscheidung findet nach dem Verfahren der Reduktionsabscheidung, Kontaktabscheidung oder durch alternierendes Aufbringen der Reduktionsmittel-und Metallsalz-Lösung aus vergleichsweise konzentrierten Lösungen und nachträglich auch nach dem Verfahren der selektiven Tampongalvanisierung statt.

Diese Verfahren, dazugehörigen Geräte und Vorrichtungen ermöglichen das Aufbringen von Metallen auf Zähnen und Knochen. Sie weisen jedoch nur eine kleine Abscheidungsgeschwindigkeit auf, sind damit sehr zeitintensiv, sind aufwendig in der Handhabung, stellen eine Gefahr für den Patienten dar, erfordern Übung und das besondere Geschick des Behandlers, sind für Patient und Behandler belastend und machen die Metallaufbringung an in vivo schwer erreichbaren Stellen nahezu unmöglich.

Aus der Kunststoff-Galvanotechnik und Spiegelherstellung sind zahlreiche Geräte zum Auftragen von Metall auf nichtleitenden Werkstoffen bekannt (Reduktionsabscheidung, Silberspritzverfahren, Leitlackauftragung usw.).

Für die Metallauftragung auf metallische Unterlagen werden beispielsweise in der Leiterplattentechnik Geräte und Verfahren für selektive Tampon- und Hochgeschwindigkeitsgalvanisierung eingesetzt.

Diese und die bisherigen Geräte zum Aufbringen von metallischen Stoffen erlauben jedoch nicht oder nur unzureichend das Aufbringen auf Zähnen oder auf Zähnen befindlichen körperfremden Materialien im Mund des Patienten, selbst wenn sie in Miniaturausführung und in Form eines zahnärztlichen Instrumentes gestaltet würden.

Es ist insbesondere für das eingangs genannte neue Behandlungsverfahren erforderlich, ein Gerät zu schaffen, das den speziellen Erfordernissen eines zahnärztlichen Instrumentes entspricht.

Die Erfindung vermeidet die Nachteile des Standes der Technik.

Der Erfindung liegt die Aufgabe zugrunde, ein einfach herstellbares, gut handhabbares zahnärztliches Werkzeug zu schaffen, mit dem sich im Mund des Patienten Metall auf Zähnen oder auf den Zähnen befindlichen körperfremden Materialien festhaftend, in kürzester Zeit, mit minimaler Gefahr für den Patienten, auch an schwer zugänglichen Stellen und in einfacher Weise aufbringen läßt.

Diese Augabe wird bei einer gattungsgemäßen Einrichtung durch die kennzeichnenden Merkmale der Ansprüche I.1, II.1 und III.1 gelöst.

Alle durch diese drei Hauptansprüche gekennzeichneten Geräteausführungen ermöglichen in nahezu gleicher Ausführungsqualität die Lösung der der Erfindung zugrunde liegenden Aufgabe, durch die effiziente Nutzung eines Metall-Abscheidungssystems bezüglich seiner Abscheidungsgeschwindigkeit und der Qualität des aufgebrachten Metalls, durch Ausschaltung auf die Abscheidungsreaktion störend wirkender Einflüsse und unerwünschter Nebenreaktionen und durch Aufrechterhaltung der optimalen Abscheidungsparameter (-bedingungen) während des Abscheidungsvorgangs.

Die hohe und gleichbleibende Abscheidungsgeschwindigkeit und Abscheidungsqualität, die einfache Handhabung und vergleichsweise hohe bzw. nahezu 100%ige Metallausbeute des Gerätes machen das Metallauftragungs-Verfahren einfach, zuverlässig und schnell durchführbar, kostengünstig und setzt die Belastungen und Gefahren für Patient und Behandler auf ein Minimum herab. Es läßt sich dadurch mit entsprechend vergrößerten Abmmessungen auch für den medizinischen Einsatz besonders vorteilhaft verwenden.

Weiterhin ist die kombinierte Verwendung der durch die Hauptansprüche gekennzeichneten Geräte in Abhängigkeit von der Objektoberflächengestalt, der Handhabung und vom abzuscheidenen Metall vorteilhaft.

Die im Anspruch I.1 gekennzeichnete Möglichkeit der Auftragung des metallischen Stoffes dient insbesondere der Verwendung instabiler Lösungen mit entsprechend hohem Reaktionspotential nach der Zusammenführung. Sie hat den Vorteil, daß das Metall sehr schnell und mit hoher Haftfestigkeit aufgebracht wird, ohne daß eine zuvor aufgebrachte Leitmetallschicht oder elektrische Anschlüsse notwendig sind. Besonders vorteilhaft wirkt sich die initial überproportionale (sonst lineare) Abscheidungsmenge in Abhängigkeit von der Auftragungsdauer aus, so daß die ungeschützte Zahnoberfläche, insbesondere im Bereich vom Dentin oder Zementum, nur für ein äußerst kleines Zeitintervall mit der Lösung in Kontakt steht, bis die Schutzwirkung durch den Metallbelag selbst eintritt.

Durch die ständige Zuführung der für sich stabilen Komponenten der instabilen Lösung wird nicht nur die Bildung der reaktiven Lösung in unmittelbarer Nähe der Objektoberfläche, eine hohe Abscheidungsgeschwindigkeit, eine hohe Metallausbeute, eine vergleichsweise hohe Haltbarkeit und einfache Bevorratung ermöglicht, sondern auch die zur Aufrechterhaltung der optimalen Abscheidungsbedingungen notwendige Beseitigung der zuvor aufgetragenen nun verbrauchten Lösung, quasi durch einen Spüleffekt gewährleistet und gleichzeitig die Gefahr für Patient und Behandler auf das Minimum herabgesetzt, da die innerhalb eines Zeitintervalls im Arbeitsfeld befindliche Menge der reaktiven Lösung im Vergleich zu den bisherigen Verfahren äußerst klein ist und, beispielsweise während einer Notabschaltung des Gerätes, umgehend reagiert und an Reaktivität verliert. Sofern die Zuführung des Abscheidungsmittels substanziell ist, beispielsweise eines Reduktionsmittels, wird die Gefahr zusätzlich dadurch herabgesetzt, daß in diesem Falle das Redoxpotential zwischen dem Metall oder dem Reduktionsmittel und den etwaig gefährdeten Strukturen beträchtlich kleiner ist als das zwischen den beiden Komponenten.

Neben der Redoxreaktion kann die Abscheidung des Metalls auch durch Reaktionen mit anderen Bestandteilen der Komponenten [Lösungsmitteln (Detergentien); Komplexliganden] oder durch Energie thermisch (Licht, Wärme), mechanisch (Ultraschall) stattfinden oder begünstigt werden mit der Folge der Überführung der Metall enthaltenen Lösung in den instabilen Zustand.

Die Metallauftragung kann in verschiedenen modifizierten Ausführungsformen des Gerätes, gekennzeichnet im Anspruch I.2, nach Art der Auftragung stattfinden. Diese Ausführungsformen tragen die genannten Vorteile in sich.

Eine besonders effektive Vermischung der Komponenten ergeben die Aerosolverfahren. Bei der pneumatischen Aerosolbildung sollte im Unterschied zu den in der Technik eingesetzten Geräten die Aerosolqualität und ihre Austrittsgeschwindigkeit der kleinen Arbeitsentfernung entsprechend schon unmittelbar nach dem Austritt aus den Düsen, die zur Abscheidung optimalen Werte aufweisen (vergleichsweise große Gasaustrittsfläche, kleine Drücke, kleine Flüssigkeitszuführungsraten).

Eine besonders preisgünstige Ausführung stellt ein mit Treibgas betriebenes Sprühgerät mit vorzugsweise über einen Schlauch an den Druckbehälter angeschlossenen Behandlungsschaft dar.

Die Ultraschallzerstäubung hat neben einer platzsparenden Ausführrbarkeit am Funktionsende und einfachen Steuerbarkeit zusätzlich den Vorteil, die Flüssigkeitstropfen in einen aktivierten Zustand mit Einfluß auf das Reaktionspotential zu überführen. Der Ultraschall kann aber auch ausschließlich zur Aktivierung ohne Zerstäubung eingesetzt werden. Besonders vorteilhaft wirken sich auch die Kombinationen der verschiedenen Auftragungen aus. So bewirkt beispielsweise die Kombination von Ultraschall oder hydraulischer Zerstäubung und Druckluft eine einfach veränderlich einstellbare Aerosolbildung mit unterschiedlichen Zusammensetzungen, Geschwindigkeiten unabhängig voneinander.

Weiterhin ist es vorteilhaft, die Aerosolbildung mehr als einmal nacheinander oder vor dem Ende der Auftragvorrichtung durchzuführen. So läßt sich eine Komponente in einer zwischengeschalteten Zerstäubungskammer, vorzugsweise mit Ultraschall oder pneumatisch betrieben, in ein Aerosol überführen, das dann mittels Unter- oder Überdruck zur Austrittsöffnung der Auftragvorrichtung transportiert wird und hier an der Düse eine weitere Zerstäubung erfährt oder bei besonders kleiner Ausführung nur weitergeleitet wird.

Die Vermischung der Komponenten kann auch unter Ausnutzung der Trägheit eines Aerosols bei alternierender oder rotierender Bewegung stattfinden.

Es besteht auch die Möglichkeit, die Zerstäubung unmittelbar nach der Zusammenführung der Komponenten durchzuführen, womit kleinste Arbeitsentfernungen erreicht werden können. Eine im kleinen Zeitintervall alternierend durchgeführte Auftragung der Komponenten auf die Oberfläche ermöglicht auch eine Aufbringung in kleinsten Ausführungen, so beispielsweise bei der Wurzelkanalbeschichtung mit rotierender oder schwingender Bewegung, durch welche die Lösungen nacheinander aufgebracht werden (III.4).

Weiterhin kann es vorteilhaft sein, eine Energiequelle in Form einer Licht- oder Wärmequelle als Abscheidungsmittel oder zur Einflußnahme auf das Reaktionspotential einzusetzen. So läßt sich beispielsweise auch Chrom aus seinen metallorganischen Verbindungen (Karbonylkomplex in Benzol) ohne die Notwendigkeit einer Stromquelle aufbringen. Die Energiezuführung muß dabei nicht immer direkt zur Lösung erfolgen, sondern kann auch indirekt, beispielsweise über die Erwärmung der Objektoberfläche stattfinden, womit gleichzeitig die Abscheidung auf die Oberfläche lokalisiert wird.

Besonders vorteilhaft ist es, die Auftragvorrichtungen der Ausführungen in ihren Zuführungen, als Parameter der Auftragungsart, verändern zu können, so zum Beispiel ihre Flüssigkeits- und Gaszuführungen über eine Vorrichtung zur Druckerzeugung oder die Intensität der Energiezuführungen über ein Potentiometer.

Weist eine zuzuführende Substanz bzw. Lösung einer Auftragvorrichtung eine vergleichsweise geringe Haltbarkeit (Minuten/ Stunden/ Tage) auf, ist es zweckmäßig, diese erst kurz vor Behandlungsbeginn in dem Behälter der Auftragvorrichtung anzumischen (III.5). Hierzu kann der Behälter für einen Anschluß an weitere Behälter mit den Komponenten des Gemisches versehen sein oder an eine Vorrichtung zur Aufnahme einer Kartusche, Patrone oder Mischkapsel mit den Komponenten angeschlossen sein.

Die Arbeit des Zahnarztes kann weiter vereinfacht werden durch die im Anspruch I.3 genannten Merkmale. Dies sind einerseits Möglichkeiten der schnellen Abführung der Auftragungen, insbesondere der Aerosole, aus dem Arbeitsfeld, eine Temperatureinstellung der Zuführungen oder ihrer einzelnen Komponenten, womit neben der allgemeinen Wirkung der Temperatur gleichzeitig ein Einfluß auf die Abscheidungsreaktion im Sinne der lokalisierten Reaktionspotentialänderung noch innerhalb des Gerätes vor der Zusammenführung genommen werden kann, und andererseits, Geräteausstattungen, die das Behandlungsverfahren in der Handhabung einfacher machen und optimieren. So wird dem Behandler eine bessere Sicht und Kontrolle über die Abscheidungslokalisation und Arbeitsentfernung, beispielsweise mittels Auflage, Distanzhalter oder sich kreuzender Lichtstrahlen im Arbeitsfeld, ermöglicht und das Erreichen aller Stellen der kompliziert gestalteten Kavität in vergleichsweise wenigen Zügen vereinfacht. Ein Steuer-Regelkreis mit Anschluß an Meß- und Sender-Empfänger-Vorrichtungen einerseits und an den Vorrichtungen zur veränderlichen Einstellung der Auftragungsparameter andererseits ermöglicht die automatische Anpassung an die durch die Unzulänglichkeit der menschlichen Hand bedingten Abweichungen und gleichzeitig eine Informationsübertragung.

Weiterhin kann eine lokalisierte Abscheidung durch einen gemeinsamen Funktionskopf realisiert werden (III.3).

Die galvanische Metallabscheidung, insbesondere nach dem Verfahren der selektiven Tampon- und Hochgeschwindigkeitsgalvanisierung stellt eine weitere Möglichkeit der Metallaufbringung auf die, zumindest mit einer Leitmetallschicht versehene, Zahnoberfläche dar. Sie kann zum Beispiel zur Verstärkung des Metallbelags, zum Aufbringen von Metallen und Legierungen mit besonders hohen Qualitätsansprüchen, die sich chemisch nur schwer abscheiden lassen, eingesetzt werden, und ermöglicht durch Umpolung auch eine gezielte Oxidation der Metalloberfläche beispielsweise zur Passivierung.

Die in diesem Verfahren notwendige relative Anoden-Kathoden-Bewegung ist mit den bislang bekannten Geräten für die Metallabscheidung im Mund des Patienten aufgrund des kleinen Platzangebotes und der komplizierten Gestalt der Objektoberfläche nur unzureichend möglich.

Die Erfindung schafft nun auch, wie in Anspruch II.1 gekennzeichnet, ein gut handhabbares zahnärztliches Werkzeug, mit dem es möglich ist, im Mund des Patienten Metall mit hoher Geschwindigkeit und optimalen Materialeigenschaften nach dem Verfahren der Tampongalvanisierung in einer dem Zahnarzt gewohnten Weise aufzubringen.

Die Bewegungsvorrichtung gewährleistet die für optimale Abscheidungsbedingungen notwendige relative Anoden-Kathodenbewegung und bewirkt die Elimination der Unzulänglichkeit der menschlichen Hand. Dies stellt für den Behandler nicht nur eine erhebliche Entlastung dar, der nur noch die Anlagerung an die Objektoberfläche zu bewerkstelligen hat, son dern bewirkt gleichzeitig die zuverlässige, exakt einstellbare, in einfacher Weise änderbare und steuerbare Anoden-Kathoden-Bewegung, die bei festgelegter Spannung respektive Stromdichte einen Mindestwert nicht unterschreiten darf, um sogenannte Verbrennungen vermeiden und hochqualitative Abscheidungsergebnisse gewährleisten zu können. Ebenso können dadurch Bewegungsgeschwindigkeiten, insbesondere in Anbetracht der kleinen verwinkelten Flächen, erreicht werden, die sich manuell nicht durchführen ließen. Dies ermöglicht wiederum höhere Stromdichten und Abscheidungsraten. Weiterhin kann durch die exakt einstellbare Bewegungsgeschwindigkeit auch Einfluß auf die Struktur und Eigenschaften des Metallniederschlags Einfluß genommen werden (Gefüge/ Korngröße). Die additive Bewegung durch den Behandler ist vergleichsweise klein und kann vernachlässigt werden.

Die länglich flächig (faden-, bandförmig) gestaltete Anode ermöglicht, insbesondere zur Beschichtung der approximalen Zahnoberflächen, ihre manuell einfach durchführbare, exakte und gleichmäßige Anlagerung an die kathodische Objektoberfläche während des Bewegungsvorgangs durch den Behandler, die eine Voraussetzung für eine gleichmäßig verteilte Geschwindigkeit und Qualität der Metallauftragung darstellt. Die relative Anoden-Kathoden-Bewegung ist bei diesem Gerät durch eine vergleichsweise große Weglänge der hin- und hergehenden Bewegung in ihrer Mindestgeschwindigkeit und Gleichmäßigkeit für den Behandler manuell in einfacher Weise durchführbar. Kleine Anodenschichtdicken ermöglichen weiterhin die für die benachbarten Strukturen (Trennvorrichtung/ Zahnfleisch) besonders schonende Durchführung der Metallauftragung.

Auch bei diesen Ausführungsmöglichkeiten der Erfindung wird die Aufrechterhaltung der optimalen Abscheidungsbedingungen erreicht. Denn die optimale Anoden-Kathodenbewegung gewährleistet nicht nur die Elimination der sich auf der Objektoberfläche bildenen Gase und Substanzen, sondern bewirkt gleichzeitig den Austausch der in unmittelbarer Nähe der Abscheidungsoberfläche vorliegenden, durch die Abscheidungsreaktion verbrauchten, Lösung mit der in der Ummantelung gespeicherten noch frischen Lösung.

Die im Anspruch II.2 gekennzeichneten Ausstattungen und Gestaltungsmöglichkeiten des Gerätes vereinfachen die Handhabung, machen die Auftragung sicher, patientenfreundlich, schnell, gleichmäßig, zuverlässig, kostengünstig und ermöglichen die Metallauftragung auch an schwer erreichbaren Stellen und bei kleinstem Platzangebot.

Die rotierende Anode, die auch in der Technik zur Beschichtung an großen Objekten Anwendung findet, trägt den Vorteil in sich, daß die Anoden-Kathodenbewegung gleichförmig stattfindet und so die Elimination und der Elektrolytaustausch durch den anlagerungsbedingten Umwälzungsvorgang besonders effektiv ist. Dies trifft besonders für die parallel zur Rotationsachse gelegenen Flächen zu.

Ein besonders vorteilhafter Wirkungseffekt wird durch die Kombination des rotierenden Antriebs und der Gestaltung der rotationssymmetrischen Anode mit ihrer Ummantelung in Form des zur Präparation des selben Flächenabschnittes verwendeten Schleifinstrumentes entsprechend erzielt. Damit wird durch die Anlagerung der Anode mit entsprechender Neigung die Objektoberfläche in Form eines Streifens über ihre gesamte Ausdehnung in dieser Orientierung benetzt, d.h. beschichtet. Der Behandler kann nun, in einer ihm besonders gewohnten Weise, die Anode gemäß der Handhabung des entsprechenden Schleifinstrumentes, bei der kurz zuvor von ihm selbst durchgeführten Präparation, in einem Zug über diesen Flächenabschnitt führen und damit beschichten. Deshalb ist es auch zweckmäßig, das Handstück mit der Anode und Bewegungsvorrichtung den gebräuchlichen Winkelstücken der zahnärtlichen Praxis gemäß zu gestalten.

Die hierdurch erzielten Vorteile beschränken sich jedoch nicht nur auf die einfache und gewohnte Handhabung, sondern bestehen ebenso in der gleichmäßigen und zuverlässigen Auftragung, insbesondere in den zervikal gelegenen Bereichen, die mit den herkömmlichen Geräten nur schwer erreicht und entsprechend weniger beschichtet werden.

Ein weiterer wesentlicher Vorteil dieser Ausführungsmöglichkeit wird durch die Gestaltung der Anode in abweichender Form oder in Form der zu beschichtenden Teiloberfläche in einer Orientierung entsprechend, beispielsweise zur gleichzeitigen Beschichtung der geringfügig über die Präparationsfläche hinausgehenden Fläche, erreicht. Bei einer Stufenpräparation würde eine zylindrische Anode an ihrem Ende eine verjüngte Weiterführung in ebenfalls zylindrischer oder umgekehrt kegelförmiger Form aufweisen. Damit läßt sich zusätzlich der über die Präparationsgrenze hinausgehende Belag in seiner Breite einfach und gleichmäßig festlegen.

Auch Wurzelkanäle können mit dieser Ausführungsmöglichkeit effektiv beschichtet werden. Um eine einfachere und sichere Handhabung und kontrollierte Abscheidung zu erhalten, kann es vorteilhaft sein, die Beschichtung schrittweise durchzuführen. Hierzu ist es zweckmäßig, die Anoden nur im Bereich ihrer Spitze oder ihres Endabschnittes mit der Anodenfläche und Ummantelung zu versehen. Damit kann auch die apikale Region des Kanals zuverlässig beschichtet werden. Mittels eines Anoden-Sets kann dann die Wurzelkanalwandung durch Einsatz der nächst höheren Größe von apikal nach zervikal vollständig beschichtet werden. Eine solche Anode kann auch dazu dienen, die Arbeitslänge beispielsweise vor der Beschichtung oder Abfüllung besonders exakt und non-invasiv zu bestimmen.

Eine weitere besonders vorteilhafte Ausführungsmöglichkeit ist es, die Anode in einer hin- und hergehenden Schwingung (Bewegungsvorrichtung) zu bewegen. Die hiermit verbundene ungleichförmige Anoden-Kathoden-Bewegung macht sich mit Kleinerwerden der Bewegungsamplitude bzw. -weglänge bemerkbar und kann über die Steuer-Regelvorrichtung durch die Steuerung der Spannungsquelle kompensiert werden. Flächig gestaltete Anoden, beispielsweise blatt-, scheiben- oder streifenförmig, ermöglichen in einfacher Weise auch die Beschichtung besonders schwer erreichbarer Stellen, wie zum Beispiel die Approximalflächen im Interdentalraum und nach Separation sogar die Flächen des Kontaktpunktes oder von okklusal betrachtet unter sich gehende Stellen, wie die über die Präparationsgrenze hinaus gehende Fläche und anderen, auch für die natürliche und künstliche Zahnreinigung nicht oder nur schwer zugänglichen und damit, aus karies-und sekundärkariesprophylaktischer Sicht, besonders gründlich zu versorgenden Bereichen. Die durch die dünne Gestaltung auftretende Flexibilität kann dabei auch zum Erreichen unter sich gehender Stellen vorteilhaft sein, so daß die Anode sich durch die Federwirkung diesem Abschnitt anlegt.

Eine der wesentlichen und bedeutsamen vorteilhaften Austattungen des Gerätes stellt die Vorrichtung zur Widerstandsmessung zwischen den Abscheidungselektroden mit dem Anschluß an die Steuer-Regelvorrichtung und das Informationsübertragungssystem dar. Der Widerstand zwischen Anode und Kathode verhält sich umgekehrt proportional zur Effektivität der Anodenanlagerung. Die optimale Anlagerung ist durch den individuell minimalen Widerstandswert gekennzeichnet. Eine nicht parallele Anlagerung ergibt durch Verkleinerung der Anoden-Kathoden-Fläche eine Zunahme des Gesamtwiderstands, jedoch einen unveränderten oder eher verkleinerten Wert an der noch vorliegenden Anlagerungsstelle. Eine konstant eingestellte Spannung führt deshalb zu einer erhöhten Stromdichte an dieser Stelle mit der Folge verschlechterter Abscheidungsqualität. Um dies vermeiden zu können, muß die Spannung mit einem beträchtlichen Sicherheitsabstand zum Maximalwert und damit geringer Abscheidungsrate eingestellt werden.

Durch die Steuerung der Spannung in Abhängigkeit des Widerstandswertes können abscheidungsbedingte Überschreitungen der maximalen Stromdichte vermieden und gleichzeitig die Nutzung hoher Stromdichten und Abscheidungsraten mit minimaler Gefahr von Verbrennungen realisiert werden. Die weiteren Parameter mit Einfluß auf den Widerstandswert werden durch Meßvorrichtungen an den entsprechenden Stellen, wie in den Zu- und Abführungsleitungen, im Ummantelungsmaterial, und individuelle Sollwerteinstellung vor Abscheidungsbeginn im Steuer-Regelkreis registriert und ständig nachkontrolliert.

Besonders vorteilhaft ist es, die Anode aus mehreren Anodenteilflächen mit je einem Anschluß an eine Spannungsquelle mit zusammengefaßter Kathode (elektrische Schaltung) zu gestalten. Bei rotierenden Anoden werden dabei die Teilanoden vorzugsweise rotationssymmetrisch ring- oder bandförmig bzw. in konzentrischen Kreisbändern parallel zur Objektoberfläche über- oder nebeneinander angelegt. Bei schwingenden flächigen Anoden ist dagegen eine streifenförmige Anordnung senkrecht zur Bewegungsrichtung vorteilhaft. Dadurch wird die zuvor genannte Schutzwirkung vor Verbrennung je nach Anodenanzahl, auch ohne Anschluß an die Steuer-Regelvorrichtung, ermöglicht. Die Kombination dieser Anode mit dem Anschluß an den Steuer-Regelkreis hat jedoch den weiteren wesentlichen Vorteil, daß über die .Aufnahme der Widerstandswerte jeder Anodenteilfläche zur Kathode dem Behandler über das Informationsübertragungssystem nicht nur die Quantität der Anlagerungseffektivität, sondern gleichzeitig auch über die Qualität, d.h. die Lokalisation der verschlechterten Anlagerungsstellen übermittelt werden kann. Damit läßt sich die Abscheidung nicht nur besonders sicher, zuverlässig und mit nahe dem Maximalwert gelegener Stromdichte und Abscheidungsrate bei optimalen Abscheidungsqualitäten durchführen. Der Behandler wird auch über Effektivität und Fehlerstellen seiner Anlagerung umgehend informiert, so daß eine Korrektur und ständige Gewißheit die optimale Handhabung schnell und leicht erlernbar machen und sich die Behandlungsdauer weiter verkürzen läßt.

Ein weiterer Vorteil besteht darin, daß die einzelnen Anodenteilflächen auch mit unterschiedlichen Stromdichten versorgt werden können, um beispielsweise den unterschiedlichen Bewegungsgeschwindigkeiten an einer Anode (radiusabhängig) gerecht zu werden.

Bei einer länglich flächig gestalteten Anode bedingt die große Weglänge der hin- und hergehenden Bewegung, im Vergleich zur Länge des zu beschichtenden Oberflächenabschnittes, daß zu einem Zeitpunkt nur ein Bruchteil der Anodenfläche der Kathodenoberfläche angelagert ist. Daher ist es besonders vorteilhaft, die einzelnen Anodenflächen [beispielsweise A;B;C] mit Anschluß an je eine Spannungsquelle weiter zu unterteilen und die hierdurch gebildeten Anodenteilflächen [$a_1$-$a_4$, $b_1$-$b_4$, $c_1$-$c_4$] der getrennt versorgten Anoden nebeneinander elektrisch isoliert anzuordnen [$a_1$,$b_1$,$c_1$,$a_2$,-$a_4$,$b_4$,$c_4$] und sie in ihrer Breite so auszulegen, daß der Abstand der Anodenteilflächen einer Anode [$a_1$;$a_2$] zumindest im Bereich der Länge der Anlagerungsfläche am Zahn liegt. Damit lassen sich die vorteilhaften Wirkungseffekte der Anodenteilung in optimaler Weise nutzen, ohne die Anzahl der getrennt versorgten Anoden erhöhen zu müssen. Besonders einfach, dünn und kostengünstig lassen sich die flächig länglich gestalteten Anoden, trotz der komplizierten Gestaltung durch Übereinanderschichtung von metallischen (Platin und -legierungen) und nicht leitenden Folien, herstellen. Die Anodenteilflächen können beispielsweise quer zur Längsachse der Anode auf die Trägerfolie geklebt und an der Rückfläche in einer der [drei] übereinandergeschichteten (isoliert) Zuleitungsfolien mit getrennter Spannungsversorgung in Kontakt gebracht werden. Anodendikken von 0.05 mm sind dabei technisch einfach zu realisieren.

Weitere vorteilhafte Austattungen des Gerätes stellen die Vorrichtungen zur Ab- und oder Zuführung, mit denen die optimalen Abscheidungsbedingungen durch Konstanz der Elektrolytkonzentration und Sättigungsgrad der Ummantelung auch langfristig ermöglicht werden und gleichzeitig kleinste Ausführungen, eine nahezu 100%ige Metallausbeute und die Vermeidung des Kontaktes der Lösungen mit dem Patienten dar (siehe auch III.1).

Spezielle Ausführungen der Anoden und ihrer Ummantelung, die sich vergleichsweise einfach und kostengünstig herstellen lassen, die Steuer-Regelvorrichtung für die Sromquelle mit Anschluß an den Meßvorrichtungen für die individuellen und alternierenden Änderungen sowie der Einsatz spezieller Spannungsarten (Impulseplating) und Umpolung zur anodischen Oxidation (Passivierung) stellen weitere effektive Ausführungsmöglichkeiten dar. Hierzu zählen auch die aus der Technik bekannten Ausstattungen wie Notschalter, Kurzschlußsicherung, Stromzähler usw..

Die Unterbringung der Anode in Form eines Drahtgeflechts oder ähnlichem in der Ummantelung ermöglicht die Verwendung kleinster Ausführungen, ein einfaches Auswechseln und durch die Elastizität der Unterlage auch eine selbständige Adaption an die Objektoberfläche mit gleichbleibendem Anoden-Kathodenabstand. Diese Ausführungsmöglichkeit trägt den weiteren Vorteil mit sich, daß insbesondere bei komplizierter Gestaltung der Anode, beispielsweise mit Zu- und Abführungen, Anodenteilung, der an die Bewegungsvorrichtung angeschlossene Schaft mit Kupplung und Leitungen durch aufsteckbare Anoden für mehrere (oder alle) Anodenformen verwendet werden kann.

Die im Anspruch III.1 gekennzeichnete Ausführung des Gerätes trägt den Vorteil in sich, daß die zur Aufbringung des metallischen Stoffes dienende, das Metall enthaltende, Flüssigkeit auf der Oberfläche des Objektzahnes in ihrer für die Abscheidungsreaktion optimalen Konzentration und Reinheit zu jedem Zeitpunkt, für eine beliebig lange Dauer, vorliegt. Dadurch wird die für ein Abscheidungssystem maximale und gleichbleibende Abscheidungsrate und hohe Abscheidungsqualität erreicht. Dies betrifft aber nicht nur die chemische Zusammensetzung, sondern auch die Temperatureinstellung.

Eine leichte und sichere Handhabung wird durch das Wegfallen des bislang erforderlichen, aufwendigen und mit Gefahren verbundenen Eintauchens des Flüssigkeitsträgers in den Behälter erreicht. Gleichzeitig wird auch die Gefahr des Kontaktes der Lösung mit den anatomischen Strukturen im Arbeitsfeld zuverlässig vermieden. So führt ein Kontakt der Lösung oder des Wattebausches mit den physiologischen Flüs sigkeiten (Speichel/ Sulkusflüssigkeit) zur unvermeidlichen diffusiven Substanzabgabe, insbesondere bei hohen Temperaturen und Konzentrationen und bei Anwesenheit von Benetzungsmitteln an die Strukturen, wohingegen beim erfindungsgemäßen Gerät der Speichel mit abgeführt wird, so daß der Kontakt ausbleibt.

Ein weiterer wesentlicher Wirkungseffekt besteht in der kleinen Ausführbarkeit, die für das Erreichen aller Stellen und für eine lokale Auftragung notwendig ist.

Bei Abscheidungen, bei denen die Lösungen in vergleichsweise instabile oder reaktive Form eingesetzt werden, wie beispielsweise bei hohen Arbeitstemperaturen, kann durch entsprechende Zusatzausstattungen die Erwärmung erst kurz vor der Auftragung durchgeführt werden, so daß die Haltbarkeit der Lösungen und ihre optimale Zusammensetzung gewährleistet wird und nur so viel Lösung überführt werden muß, wie für die Auftragung benötigt wird. Weitere Austattungen wie Kühlvorrichtungen und Vorrichtungen zur Zusammenführung der Lösungen in Form besonders stabiler Komponenten machen die Substanzdeponierung für lange Zeiträume möglich.

Weiterhin gewährleistet der Durchfluß der Lösungen einen effektiven Austausch der "verbrauchten" mit der neu zugeführten Flüssigkeit in unmittelbarer Nähe der Abscheidungsoberfläche.

Gefahren von Verunreinigungen werden hierdurch gleichzeitig auf ein Minimum herabgesetzt.

Die abgeführten Lösungen können in einem Behälter in vergleichsweise hoher Konzentration aufgefangen und entsorgungs- und wiederaufbereitungstechnisch (Edelmetalle) aufgrund der bekannten Zusammensetzung und hohen Konzentration einfach und preisgünstig weiterverarbeitet werden.

Bei Verwendung beständiger Lösungen lassen sich nahezu 100%ige Metallausbeuten erreichen. Aufgrund der kleinen abzuscheidenen Metallmenge (5-10 mg Silber/ 10-20 mg Gold) kann durch ein entsprechend großes Kreislaufvolumen (100-1000 ml) die Konzentrationseinstellung auch in vergleichsweise großen Zeitabständen durchgeführt werden.

Mögliche unterschiedliche in Bezug auf die Abscheidungs-und Auftragungsarten ausgerichtete Anordnungen und Ausstattungen des Gerätes sind im Anspruch III.2 gekennzeichnet. Dabei kann die Reduktionsabscheidung aus stabilen und metastabilen Lösungen und die Hochgeschwindigkeitsgalvanisierung vorzugsweise durch langsame Zuführung oder in Form von Spritzstrahlen stattfinden; bei der Verwendung instabiler Lösungen ist die Auftragung im Spritz- oder Sprühverfahren besonders geeignet, aber auch die langsame Zuführung, beispielsweise bei Verwendung von Energiequellen als Abscheidungsvorrichtungen oder Wurzelkanalbeschichtung, ist vorteilhaft. Die zur Beschreibung der Hauptansprüche I und II erläuterten Anordnungen und Ausstattungen sind auch auf diese Ausführungsmöglichkeit sinngemäß übertragbar zu verstehen (z.B. Steuerung der Spannungsquellen).

Die in den Ansprüchen III.3 und III.4 gekennzeichneten Ausführungsformen und Ausstattungen erleichtern dem Zahnarzt die Handhabung, verbessern den Schutz für den Patienten und ermöglichen in einigen Fällen, auf weitere Sicherheitsvorkehrungen verzichten zu können.

Der Funktionskopf mit dem Auflagekissen ermöglicht eine lokalisierte Metallaufbringung durch seine Anlagerung an die Objektoberfläche. So können hierdurch auch alle Schritte der Metallaufbringung (Ätzen, Bekeimen, Aktivieren, Spülen) lokalisiert und ohne jede weitere Sicherheitsvorkehrung durchgeführt werden (Fissuren, Grübchen, punktuelle Nachbehandlung). Das Auflagekissen muß jedoch nicht immer abdichtend wirken, sondern kann auch als Distanzhalter (z.B. Aerosolverfahren) oder zur Begrenzung der Abscheidung dienen, da schon durch die Absaugung ein Austritt der Lösungen vermieden wird. Besteht es aus einem Flüssigkeit speichernden Material, wie beispielsweise Watte, kann es gleichzeitig als Puffer für die Absaugung der Flüssigkeiten dienen. Dabei wird die Pufferkapazität durch den Anschluß an die Absaugvorrichtung aufrechterhalten. Diese Wirkungsweise gilt für alle Absaugvorrichtungen, die mit diesem Puffer in Verbindung stehen und ist besonders geeignet für die Aerosolverfahren, da hier die Aerosoltropfen schnell in der Watte aufgenommen werden und eine gewisse Durchlässigkeit für die Gaskomponente vorliegt.

Besonders vorteilhaft ist es auch, wenn die Auflagekissen doppelseitig angeordnet sind. Hiermit lassen sich in besonders sicherer und einfacher Weise die Approximalflächen zweier benachbarter Zähne beschichten. Besteht das Kissen dabei aus dem elastischen Hohlkörper, läßt es sich in einfacher Weise in den Interdentalraum einführen und nach Positionierung durch Druckerzeugung quasi aufblasen und kann gleichzeitig der Separation zur Freilegung der zu beschichtenden Flächen dienen. Durch diese Auflagekissengestaltung wird auch eine besonders effektive Anlagerung und Abdichtung erreicht. Der elastische Hohlkörper läßt sich durch Anbringung beispielsweise an die Seiten- oder Rückflächen zur optimalen Fixierung und Abdichtung eines Funktionsendes oder -kopfes einsetzen (auch beim Wurzelkanalinstrument III.4). So kann ein flächenhaft gestalteter Funktionskopf beispielsweise für die Tampongalvanisierung, der in den Interdentalraum eingeführt ist, durch sein Kissen optimal angelagert werden.

Ein Funktionskopf mit zwei unterteilten Kammern ist zum Beispiel eine besonders geeignete Ausführung für die Hochgeschwindigkeitsgalvanisierung. Denn die Kammertrennung mit der Anode (Durchflußwiderstand erhöht) bewirkt die Beschleunigung der Lösung gerade im Anoden-Bereich, so daß der notwendige Spüleffekt gewährleistet wird. Ein besonders vorteilhafter Effekt ist dabei, daß die Durchflußrate vergleichsweise klein, der Quotient aus benetzter Oberfläche und Flüssigkeitsmenge groß ist und die Druckerzeugung auf die Flüssigkeit durch Unterdruck in der Absaugkammer gebildet werden kann, so daß bei unexakter Anlagerung Luft angesaugt wird.

Die Aufbringung einer Leitmetallschicht oder Beschichtung von Wurzelkanalwandungen kann in einfacher Weise nach dem Verfahren der Reduktionsabscheidung aus stabilen oder meta stabilen Lösungen, aber auch durch Zusammenführung instabiler Lösungen, stattfinden.

Durch die in den Anspruch III.4 gekennzeichnete Gestaltung gelingt es, insbesondere den apikalen Teil des Wurzelkanals einfach und sicher zu beschichten. Anschluß an Steuer-Regelvorrichtungen zur Vermeidung schädlicher Druckbildung machen das Gerät noch zuverlässiger. Die im Aerosol- oder Spritzverfahren durchgeführte Beschichtung weist die zum Anspruch I erwähnten Vorteile auf. Dabei kann es vorteilhaft sein, die Zerstäubung in einer zwischengeschalteten Kammer stattfinden zu lassen und das Aerosol beispielsweise mittels des Gas-Strahlpumpen-Effektes zur Austrittsöffnung zu transportieren. Ihre spaltförmige Anordnung ermöglicht die Beschichtung in wenigen Schritten und begünstigt die effektive Vermischung der Komponenten auf der Oberfläche. Eine zur Wurzelkanalöffnung gerichtete Auftragungsrichtung erleichtert die Abführung und ermöglicht vergleichsweise hohe Abscheidungsraten. Insbesondere beim Spritzvorgang wird die effektive Vermischung durch die Bewegung unterstützt bzw. gewährleistet.

Das Tauchverfahren stellt eine weitere vorteilhafte Aufbringungsart dar und ermöglicht eine vollständige gleichzeitige, gleichmäßige und exakt berechenbare Metallbelegung.

Die mechanisch betriebene Vorrichtung zur Druckbeaufschlagung für die Zu- und Abführung der Stoffe trägt, neben den offensichtlichen Vorzügen, den Vorteil in sich, daß die Volumentransportraten exakt und zuverlässig festgelegt, gesteuert und einfach in den Steuer-Regelkreis aufgenommen werden können und die durch unvorhergesehene Zwischenfälle mit Einfluß auf den Flüssigkeitstransport auftretenden Druckänderungen im Gerät selbst und im Arbeitsfeld durch Druckmessung in Quantität und Qualität (zeitlicher Verlauf) in einfacher Weise (Piezoeffekt) exakt aufgenommen werden können. Die damit verbundenen Gefahren für Patient und Behandler können so über den Steuer-Regelkreis einfach und zuverlässig vermieden werden.

Weitere in den Ansprüchen III.5 bis III.7 gekennzeichnete Ausführungen des Gerätes ermöglichen insbesondere eine einfache Verwendung, Wartung und einen sparsamen Umgang mit den Lösungen. Die Verwendung von Trennvorrichtungen ist zwar mit einem erhöhten Aufwand verbunden, macht die Auftragung mit dem Gerät jedoch besonders sicher, einfach und unempfindlich hinsichtlich Verarbeitungsfehlern. Besonders vorteilhaft ist dabei der Einsatz von solchen Vorrichtungen, die die Einbringung eines chemoplastischen Materials in den Zahnfleischsulkus beinhalten und auf diese Weise die Präparationsfläche und ihre angrenzenden Flächenabschnitte, insbesondere an den schwer erreichbaren und damit klinisch wichtigen Stellen freihalten, optimal abdichten und ihre angrenzenden anatomischen Strukturen schützen.

Das Wesen der Erfindung ist nachstehend anhand von in der Zeichnung schematisch dargestellten Ausführungsbeispielen näher erläutert.

Es zeigen:

Fig. 1: Eine grobschematisch dargestellte Aufteilung des Gerätes.

Fig. 2: Ein Funktionsende für die Auftragung im Spritzverfahren in Längsschnittdarstellung.

Fig. 3: Ein Funktionsende für die Auftragung im pneumatischen Aerosolverfahren in Querschnitt- [3a] und Längsschnittdarstellung [3b].

Fig. 4: Ein Funktionskopf mit Auflagekissen für eine thermische Abscheidung in Längs- [4a] und Querschnittdarstellung [4b].

Fig. 5: Ein Funktionskopf mit Auflagekissen für die Auftragung im pneumatischen Aerosolverfahren in Längs- [5a] und Querschnittdarstellung [5b].

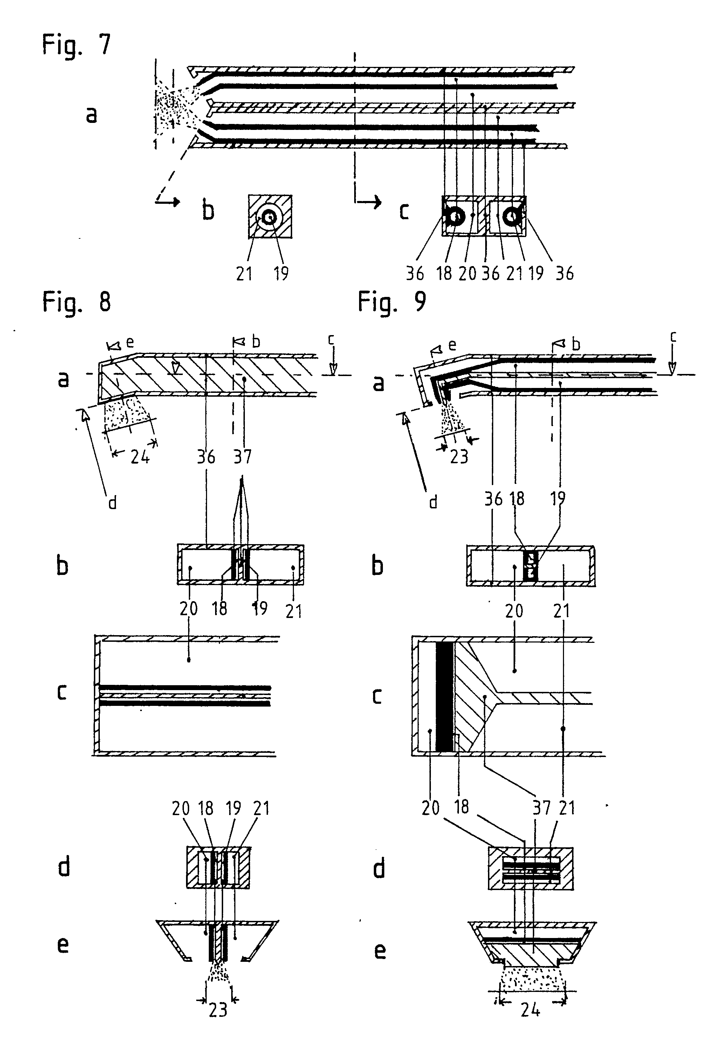

Die Figuren: 6a bis 6e zeigt zwei und die Figuren 7a bis 7c, 8a bis 8e und 9a bis 9e zeigen je ein Funktionsende für die Auftragung im pneumatischen Aerosolverfahren in unterschiedlichen Schnitten und Aufsichten, die in den Figuren dargestellt sind.

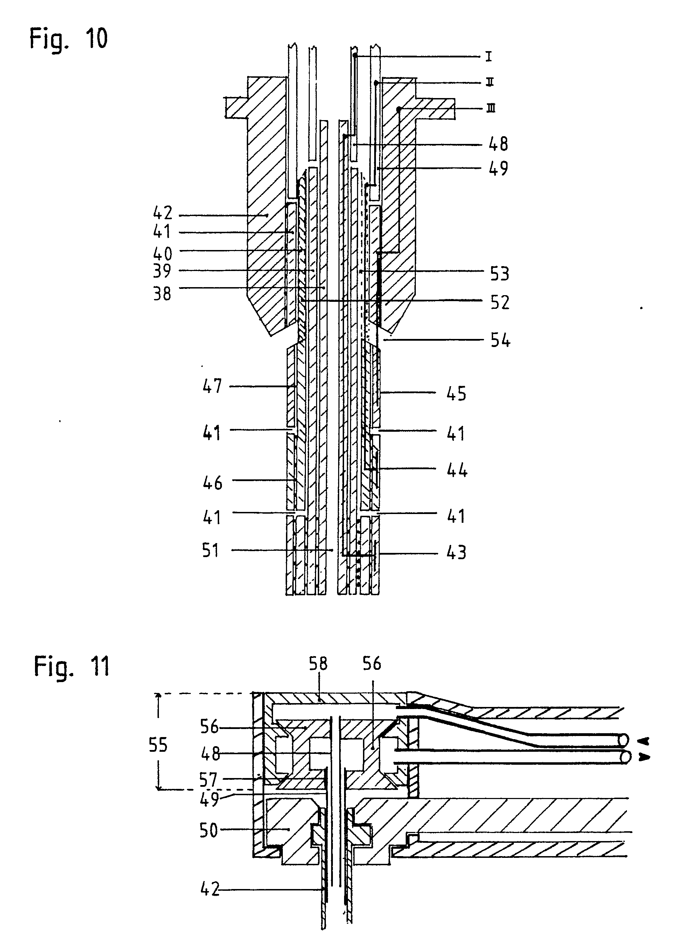

Fig.10: Eine teleskopartig aufgebaute Anode für den rotierenden Antrieb mit interner Zu- und Abführung und Anodenteilung.

Fig.11: Einen Bewegungsantrieb für eine rotierende Bewegung mit Kupplung für die Zu- und Abführung von den Flüssigkeiten.

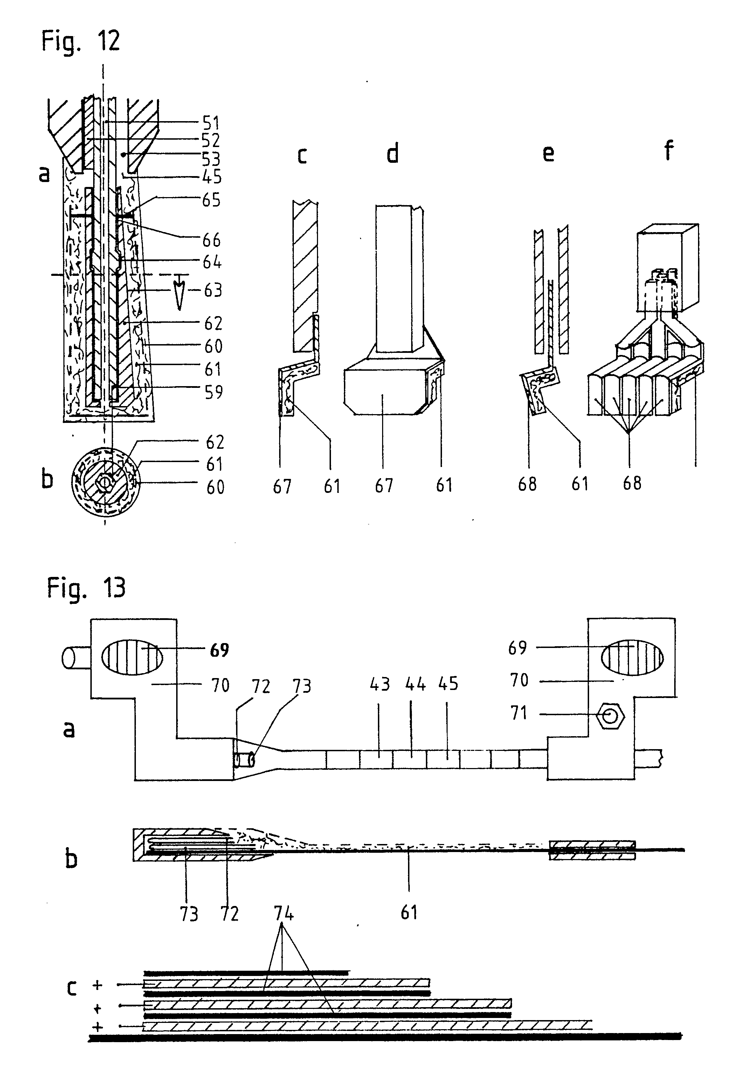

Fig.12a und b: Zwei Anodenhälften für einen rotierenden Bewegungsantrieb in Längs- [12a], in Querschnittdarstellung [12b] mit aufsteckbarer Ummantelung, in zylindrischer (li) und umgekehrt kegelförmiger (re) Gestalt;

Fig.12c und d: Eine Anode für einen schwingenden Bewegungsantrieb in Längsschnittdarstellung [12c] und Aufsicht [12d];

Fig.12e und f: Eine Anode für einen schwingenden Bewegungsantrieb in Längsschnittdarstellung [12c] und Aufsicht [12d].

Fig.13: Ein Gerät für die Beschichtung approximaler Zahnflächen für eine manuelle Bewegungsdurchführung in Aufsicht [13a] und in Längsquerschnittdarstellung auf Höhe der Anode [13b] und eine stark vergrößert dargestellte Anodenaufbauart aus Folien [13c].

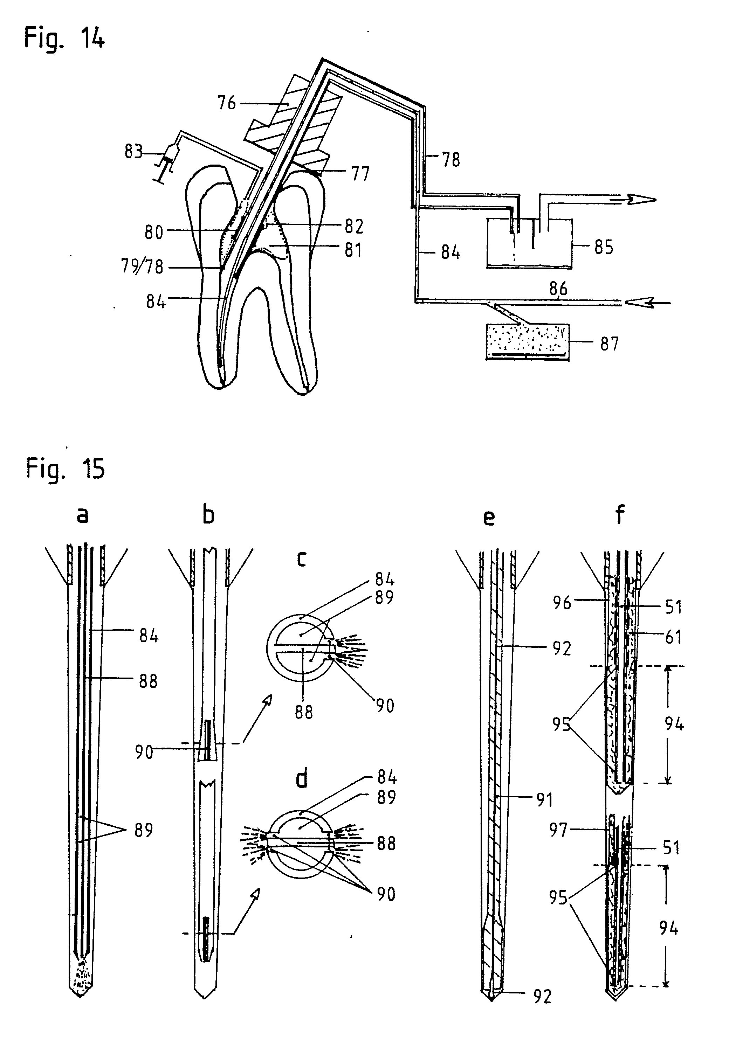

Fig.14: Eine Situationsübersicht des Gerätes für eine Wurzelkanalbehandlung.

Fig.15 a bis d: Drei im Wurzelkanal befindliche Funktionsköpfe für die Auftragung im Aerosol- oder Spritzverfahren in Längs-[15a und b] und Querschnittdarstellung [15c und d];

Fig.15 e: eine im Wurzelkanal befindliche Meßanode zur Arbeitslängenbestimmung in Längsschnittdarstellung;

Fig.15 f: Zwei im Wurzelkanal befindliche Anoden für die galvanische Metallaufbringung unterschiedlicher ISO-Größen in Längsschnittdarstellung.

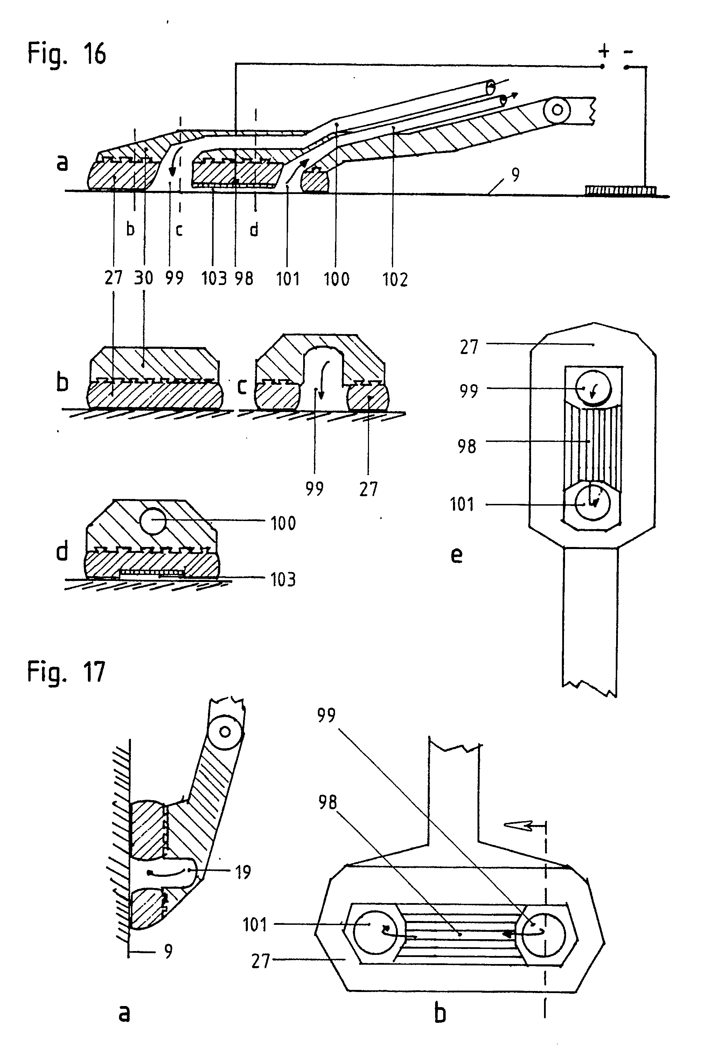

Fig.16: Ein Funktionskopf mit Auflagekissen und zweigeteilter Kammer für die galvanische Metallaufbringung in Längs- [16a] und drei bezeichneten Querschnittdarstellungen [16b bis d] und Aufsicht [16e].

Fig.17: Ein Funktionskopf, entsprechend des in Figur 17 abgebildeten mit quer zur Halteachse gelegener Durchflußrichtung in bezeichneter Querschnittdarstellung [17a] und Aufsicht [17b].

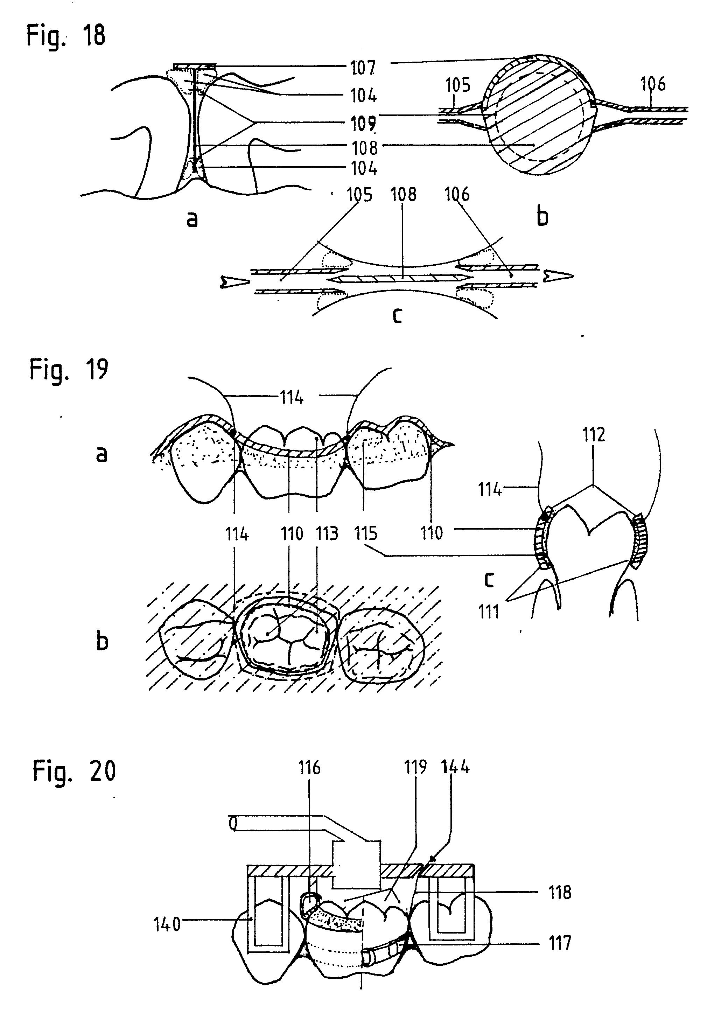

Fig.18: Ein Funktionskopf mit doppelseitigem Auflagekissen für die Einbringung in den Interdentalraum, zur Beschichtung der Approximalflächen in Querschnittdarstellungen [18a und c] und Längsschnittdarstellung [18b].

Fig.19: Eine Trennvorrichtung für die lokalisierte Beschichtung der Kaufläche eines Zahnes in vivo, in Längs-[19a] und Querschnittdarstellung [19c] und Aufsicht [19b].

Fig.20: Ein Funktionskopf mit Anbringung an eine Gestellvorrichtung mit Auflagekissen [li] oder einem elastischen Hohlkörperring mit Trennfolie [re].

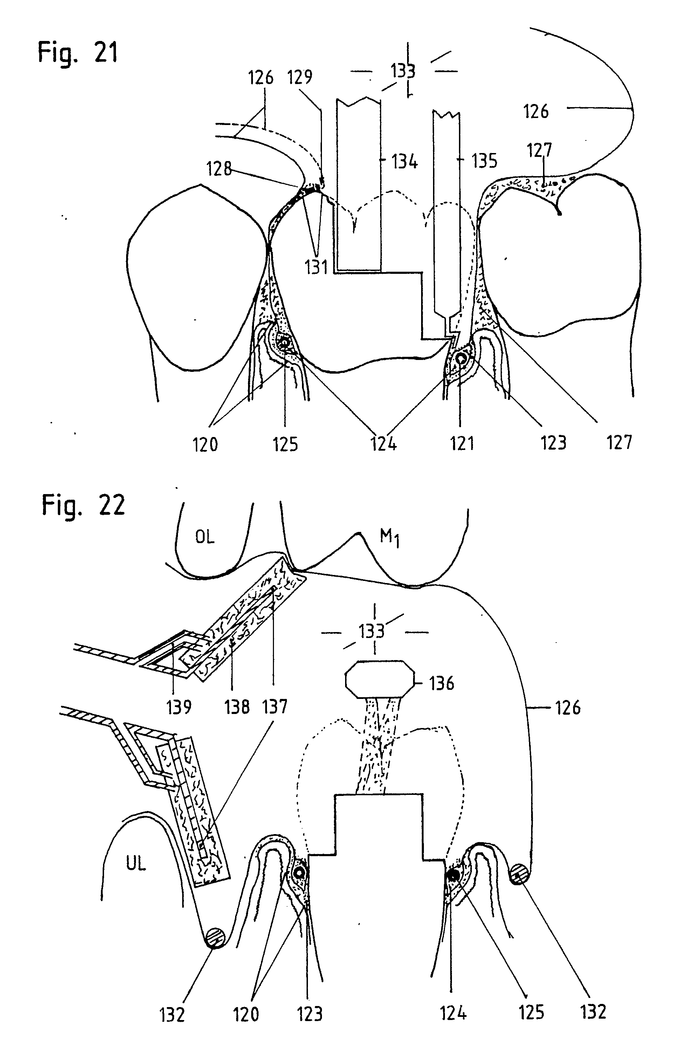

Fig.21: Die schematische Darstellung des Arbeitsfeldes mit Trennvorrichtung in vivo, in mesiodistaler Längsschnittdarstellung.

Fig.22: Die schematische Darstellung des Arbeitsfeldes mit Trennvorrichtung in vivo, in vestibulo-oraler Längsschnittdarstellung.

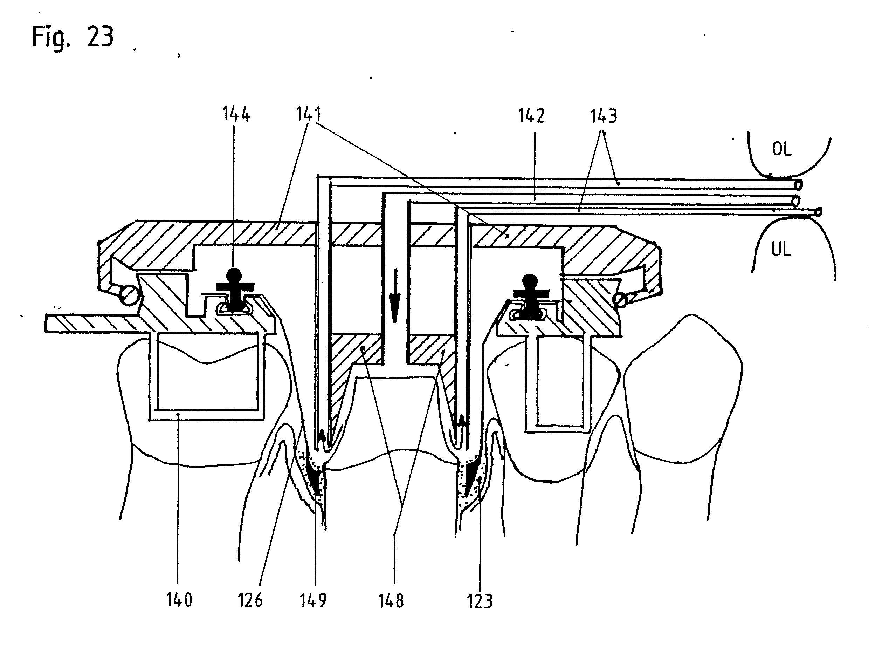

Fig.23: Einen Funktionskopf für die Abscheidung nach dem Tauchverfahren in mesio-distaler Längsschnittdarstellung.

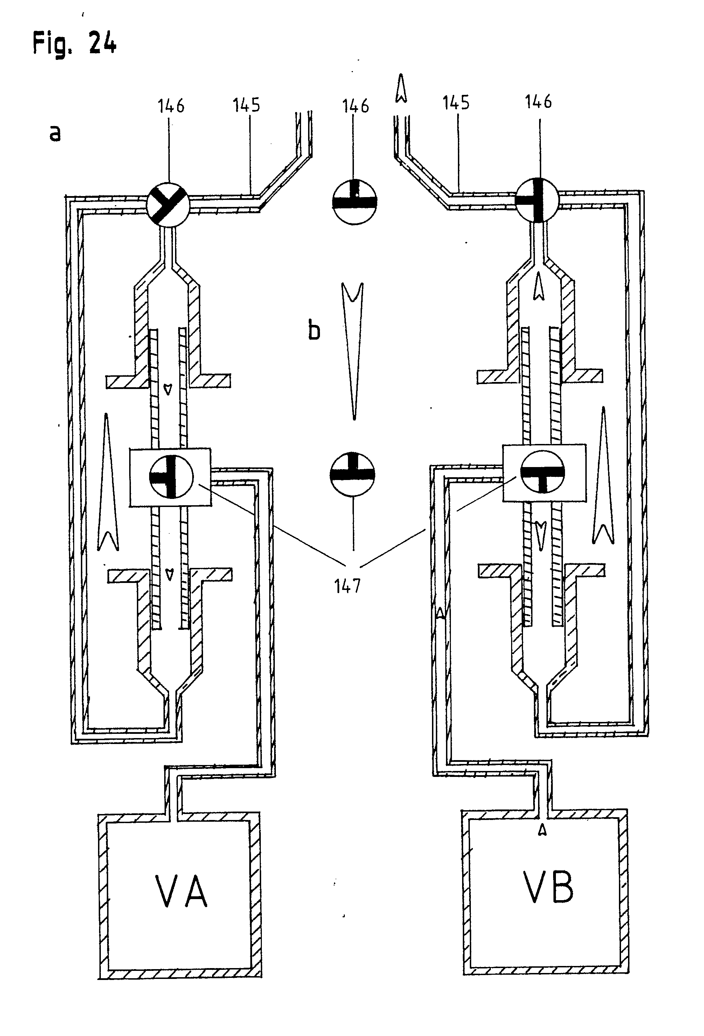

Fig.24: Zwei Flüssigkeitsbehälter für den Anschluß an Auftragvorrichtungen mit verschiedenen Schalterstellungen [24a und b].

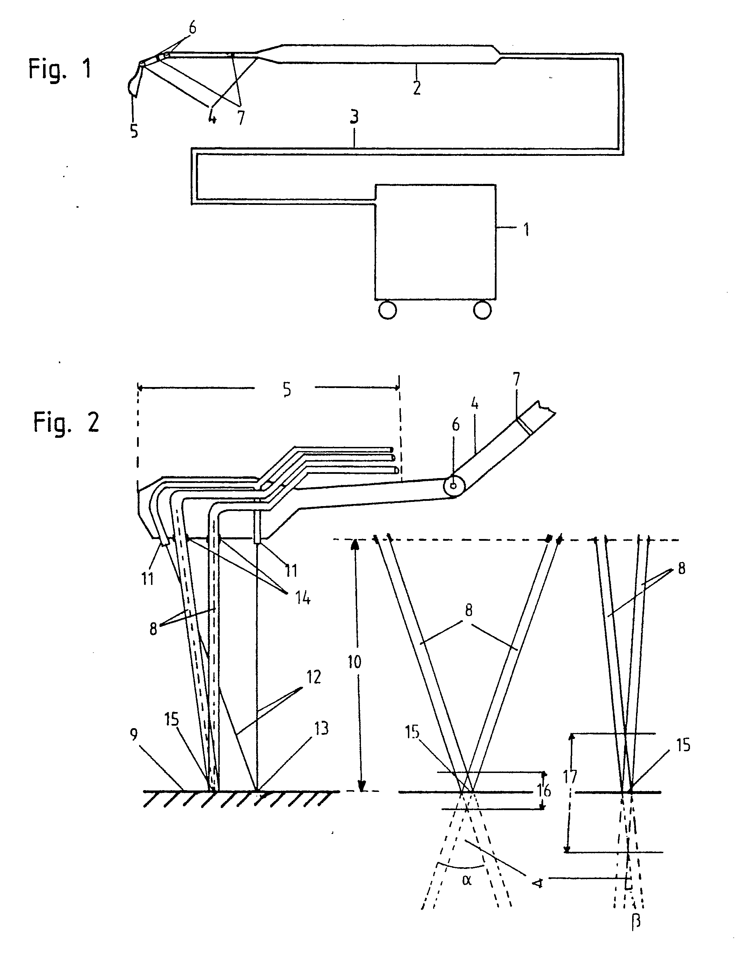

Die in Figur 1 dargestellte Aufteilung des Gerätes besteht aus einer Gerätebox (1), einem Handstück (2) mit Verbindungsschlauch (3) und dem das Funktionsende oder den Funktionskopf (5) tragenden (hier) oder bewegenden aufsteckbaren Behandlungsaufsatz (4). Durch die Scharnier- (6) und Rotationsgelenke (7) kann das Funktionsende in die unterschiedlichen Positionen, relativ zum Handstück, gebracht werden.

Der Kreuzungspunkt der aus den Spritzdüsen (14) austretenden Spritzstrahlen (8) des in Figur 2 skizzierten Funktionsendes stimmt mit dem gemeinsamen Auftreffpunkt (15) auf der Objektoberfläche (9) überein und weist die selbe Distanz (10), die sogenannte Arbeitsentfernung, von den Austrittsöffnungen (14) der Spritzdüsen wie der Kreuzungspunkt (13) der Lichtstrahlen (12) von ihren Lichtleiterenden (11) auf. Die Länge des Streckenabschnittes (16) und (17), in dem die Spritzstrahlen sich kreuzen und effektiv vermischen, nimmt mit Kleinerwerden ihres Kreuzungswinkels (alpha/beta)zu, wodurch die Empfindlichkeit der Abscheidungsreaktion auf Abweichungen von der optimalen Arbeitsentfernung bei kleinen Winkelbeträgen (beta) herabgesetzt wird.

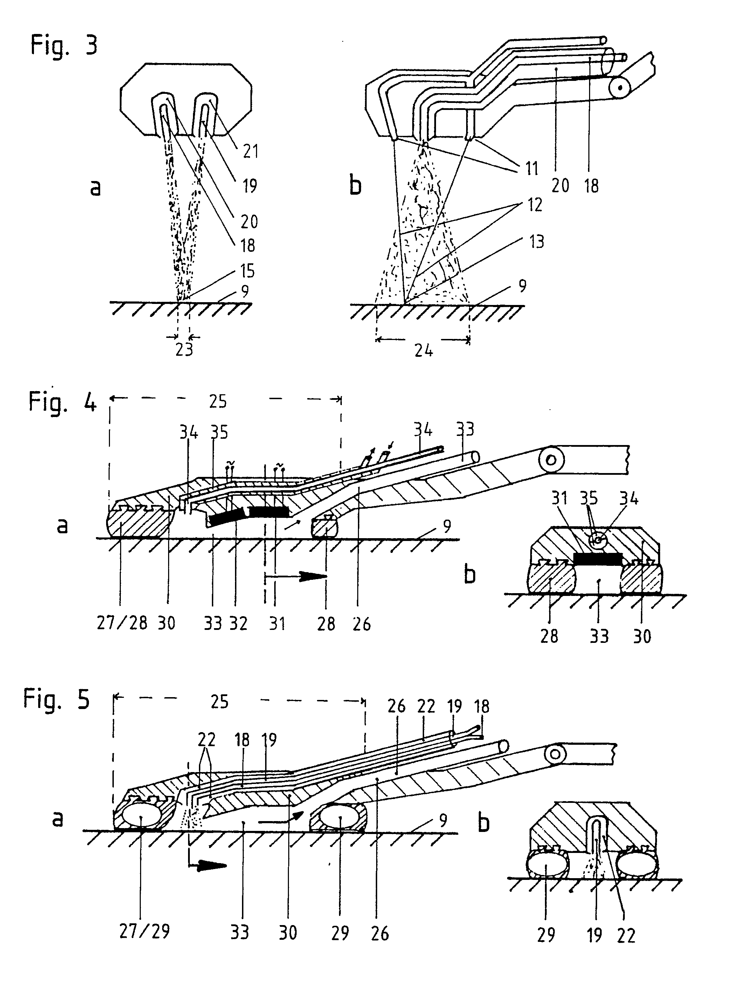

Die in den Figuren 3 und 5 bis 9 skizzierten Funktionsenden dienen der Auftragung im pneumatischen Aerosolverfahren. Sie weisen alle je eine Zuführungsleitung für die zu vermischenden Flüssigkeiten (18) und (19) und je eine Zerstäubungsgas-Zuführungsleitung (20) und (21) für beide Sprühdüsen (Fig.: 3, 6 und 7) bzw. zwei (20)/(21) Leitungen für eine zusammengefaßte Düse (Fig.: 8 und 9), oder eine zusammengefaßte Zerstäubungsgas-Zuführungsleitung (22) für beide Komponenten (Fig.: 5) auf.

Das in Figur 3a und 3b skizzierte Funktionsende erzeugt zwei Sprühstrahlen, jeweils in der Form eines Fächers, so daß die Auftrefffläche, durch die Strecken (23) und (24) im Quer- und Längsschnitt gekennzeichnet, strichförmig bzw. oval ist.

In den Figuren 4 und 5 ist das Funktionsende in Form eines gemeinsamen Funktionskopfes (25) mit Absaugrohr (26) und Auflagekissen (27), das aus gummielastischem Material (28) oder einem dünnwandigen elastisch verformbaren mit Flüssigkeit gefüllten Schlauch (29) besteht und am Gerüst (30) befestigt ist.

Der Funktionskopf der Figur 4 hat zwei Wärmequellen (31)/(32), mit gerichteter Wärmezuführung als Abscheidevorrichtung in der zentralen Kammer (33) angeordnet, die auf die Objektoberfläche bzw. durchfließende Lösung gerichtet sind. Zur Stabilisierung der thermisch instabilen Lösung ist ihre Zuführungsleitung (34) in einem Durchflußkühlrohr (35) untergebracht.

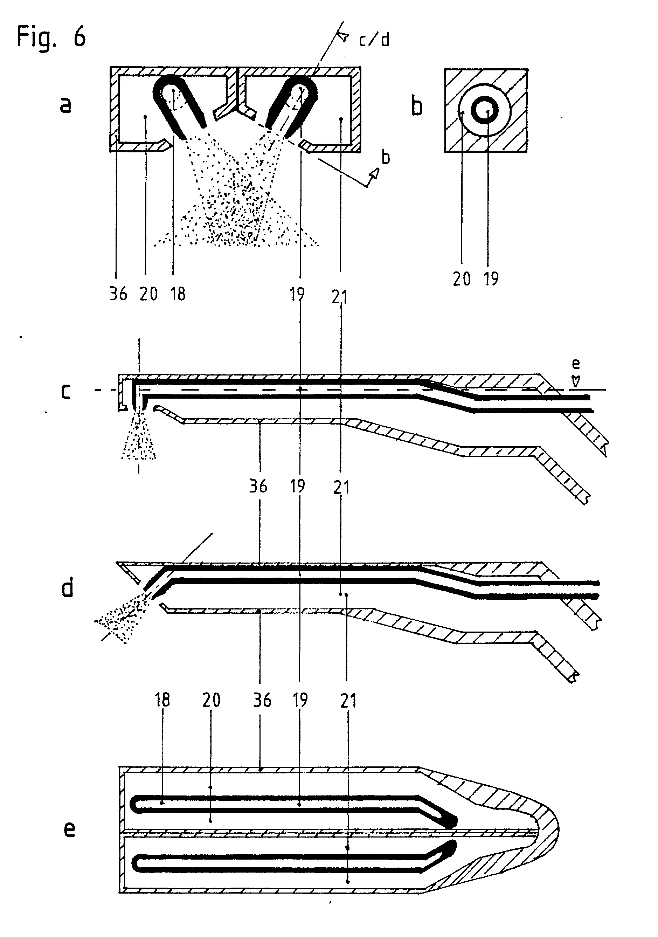

Die in den Figuren 6 bis 9 skizzierten Funktionsenden sollen als Ausführungsbeispiele die Möglichkeiten der Miniaturausführungen veranschaulichen. Hierzu können die Darstellungen in einer Maßstabswiedergabe von M = 10:1 verstanden werden, wobei Fig: 6a und 6b in doppelter Größe, M = 20:1, dargestellt wurden.

Die Funktionsenden sind aus kanülenartigen Rohren für die Zerstäubungsgas-Zuführung (36) mit den in ihren Lumen (20)/(21) untergebrachten, an der Innenwandung befestigten Zuleitungsrohren (18)/(19) oder mit Trennwänden (37) zur Lumenbegrenzung für die Zuleitungen (18)/(19) aufgebaut. Die in Figur 6 und 7 skizzierten Sprühköpfe unterscheiden sich in erster Linie durch ihre Auftragungsrichtung relativ zur ihrer Längsachse (siehe Figur 6c, 6d und 7a). Die Sprühköpfe in den Figuren 8 und 9 unterscheiden sich dagegen, bei gleicher Auftragungsrichtung, durch die Orientierung ihres fächerförmigen Sprühnebels relativ zur ihrer Längsachse (Fig. 8, parallel / Fig. 9, quer). Diese Ausführungsbeispiele eignen sich auch zur kombinierten Zerstäubung mit Ultraschall, in dem beispielsweise ihr Behandlungsaufsatz an die Ultraschallquelle angeschlossen ist (nicht gezeichnet).

Die in Figur 10 skizzierte teleskopartig zusammengesetzte Anode ist zur besseren Übersicht verzerrt [Querschnittsvergrößerung M = 20:1, Längsschnitt-Schaftanteil: M = 5:1 , -Anodenfläche: M = 10:1 ] dargestellt. Sie ist aus vier Kanülen (38), (39), (40), (41) und dem Schaftrohr (42) aufgebaut. Die drei Anodenteilflächen (43),(44),(45) werden durch die Längsteilung der äußersten Kanüle (41) gebildet und stehen durch den elektrischen Strom leitende (46) und nicht leitende (47) Verklebung oder Verbindung, mit einzelnen der inneren Kanülen und über deren elektrischen Kontakt mit der Zuführungsleitung (48), der Abführungsleitung (49) und der Aufnahmevorrichtung (50) des Bewegungsantriebs für den Schaft, per Friktion in getrennt elektrischer Verbindung (I),(II),(III) mit ihren Stromquellen. Die innerste Kanüle bildet die Zuführungsleitung (51). Der Raum zwischen der zweitkleinsten und äußersten durch Stege (52) miteinander nichtleitend verbundenen Kanülen bilden die Abführungsleitung (53) mit ihren Öffnungen (54) am Schaftübergang.

In Figur 11 ist die Kupplung (55) der rotierenden Zu- und Abführungsrohre (48) und (49), die mit dem Doppelkegel (56) mit Aussparungen für die abgesaugte Lösung (nicht gezeichnet) fest (57) verbunden ist und vorzugsweise ebenfalls direkt angetrieben wird in ihrem Gleitlager (58) dargestellt. In Figur 12a und b ist eine, auf einen Anodenhalter (59) mit Anschluß an den Bewegungsantrieb aufsteckbare Anode, deren linke Hälfte einer zylinderförmigen und rechte Hälfte einer birnenförmigen Anode zuzuordnen ist, skizziert. Die Anode selbst (60) ist als Drahtgeflecht in der Ummantelung (61) untergebracht, die mit dem vorgeformten Rahmengerüst (62) fest (63) verbunden ist. Dieses wird mittels Schnappverschluß (64) auf dem Anodenhalter gegen Abzug fixiert und von dem Anschlußdraht (65) der Anode durchsetzt, der mit dem Anodenhalter elektrisch verbunden (66) ist.

Figur 12c bis f zeigen zwei flächig gestaltete Anoden für den schwingenden Antrieb, insbesondere zur Beschichtung der Präparationsgrenze [siehe Figur 21 (135)]. Die Anode selbst besteht aus einer dünnen Scheibe (67) oder nebeneinander angeordneten plattgewalzten Kanülen (68).

Für die Beschichtung der approximalen Flächen oder spaltförmiger Räume ist die in Figur 13 dargestellte Ausführung für eine bidigitale Handhabung (69) mit zwei Anodenhaltern (70), Lösungszuführung (74) und Anodenklemmvorrichtung (71) zum Einklemmen der Anode nach ihrer Durchführung durch den Interdentalraum geeignet. Zu- (72) und Abführungsleitung (73) sind dabei an einer Seite angeordnet und werden alternierend betrieben. Eine derart dünne Anode kann auch mit Anodenteilung in einfacher Weise und kostengünstig, wie in Figur 13c grob schematisch skizziert, durch Übereinanderschichtung von dünnsten Platin- (74) und Kunststoffolien (75) hergestellt werden.

In Figur 14 ist die in vivo-Situation eines in dem Wurzelkanal eingeführten Funktionskopfes grobschematisch dargestellt. Die Haltevorrichtung (76) entspricht einem modifizierten Wurzelkanalinstrumentenhalter mit einer Referenzauflageebene (77). Das Absaugrohr (78) des Funktionskopfes ist bis zum Anschlag (79) in den Kanal bzw. Kanaleingang eingeführt und kann zusätzlich mittels des über eine Manschette (80) am Absaugrohr befestigten elastisch verformbaren, mit Flüssigkeit (81) gefüllten, Hohlkörpers (82) mit Anschluß an eine Spritze (83) abgedichtet und fixiert werden. Innerhalb bestimmter Arbeitslängenbereiche ist es auch möglich, nur die Zuführungsleitungen (84) in der Länge zu verändern. Weiterhin kann die Haltevorrichtung auch über Klemmelemente befestigt werden (nicht gezeichnet). Das Absaugrohr ist an einen Auffangbehälter (85) angeschlossen. Die Zuführungsleitung (84) ist über ein Gasrohr (86) an die ultraschallbetriebene Zerstäubungskammer (87) angeschlossen.

Figur 15a zeigt ein Spritz- oder Sprühende zur stromlosen Beschichtung, insbesondere des apikalen Bereiches, mit parallel zur Längsachse ausgerichteter Zuführungsrichtung. Das Funktionsende der Figuren 15b und c hat einen spitzen, das der Figuren 15b und d einen stumpfen Winkel seiner Zuführungsrichtung zur Längsachse. Das Funktionsende stellt ein Zuführungsrohr (84) mit zwei durch eine Trennwand (88) geteilten Lumina (89) und schlitzförmig angelegten Austrittsöffnungen (90) an seinem Ende dar. Eine effektive Vermischung der Lösungen findet bei diesem Beispiel aufgrund der kleinen Abmessungen durch die rotierende Bewegung nacheinander und nach einer (15b/c) oder einer halben Umdrehung (15b/d) wiederholend statt. Die in Figur 15d skizzierte Anordnung der Austrittsöffnungen an den gegenüberliegenden Seiten ist für eine bidigitale Bewegungsdurchführung besonders geeignet.

Die Figur 15e zeigt eine Meßanode mit isoliertem Schaft (91) und eingelagerter Drahtschlaufe (92), zum Schutz vor Beschädigung der Leitmetallschicht aus dünnstem Draht bestehend.

Figur 15f zeigt die ummantelten (96/97) Anoden mit nur in ihren Endabschnitten (94) mit festgelegter Länge angelegten Anodenflächen (95). Die obere (96) und untere (97) Anode unterscheiden sich dabei ausschließlich in ihrer Größe (ISO-Größe).

In Figur 16 ist ein Funktionskopf skizziert, der einen Querbalkenanteil mit der Anode (98) der Abscheidevorrichtung, eine Zuführungskammer (99) mit dem Zuführungsrohr (100) und eine Absaugkammer (101) mit dem Absaugrohr (102) enthält, und dessen durch die Objektoberfläche und den Querbalkenanteil gebildeter Kanal (103) eine vergleichsweise kleine Querschnittsfläche aufweist. Dieser Funktionskopf ist daher für die Hochgeschwindigkeitsgalvanisierung besonders geeignet. Figur 17 zeigt den gleichen Funktionskopf mit senkrecht zur Halteachse ausgerichteter Anordnung.

Figur 18 zeigt einen, mit doppelten Auflagekissen aus Hohlkörpern (104) mit Druckanschluß (nicht gezeichnet) versehenen, in den Interdentalraum eingefügten Funktionskopf mit Zu- (105) und Abführungsleitung (106). Das halbringförmig angeordnete Verstärkungsband (107) dient auch der einfacheren Einbringung in den Interdentalraum und ist mit der Anode (108) fest verbunden. Durch Weglassen der Anode - siehe Markierung (109) - kann dieser Funktionskopf auch für die Aufbringung nach dem Verfahren der Reduktionsabscheidung aus stabilen oder metastabilen Lösungen (Leitmetallschichtaufbringung) verwendet werden.

In Figur 19 ist eine besonders einfach aufgebaute Trennvorrichtung skizziert, die aus einer thermo- oder chemoplastisch den individuellen Konturen der Zähne angepaßten Platte (110), in Art der bekannten Kunststoff-Kappenschiene, auch mit als Halteelemente fungierende Stellen (111) besteht und ein durch den Behandler ausgespartes Loch (112) für den Objektoberflächenabschnitt (113) und die konfektioniert oder nachträglich angebrachte Trennfolie (114) aufweist. Ihre Applikation findet mittels einer chemoplastischen, elastischen und vergleichsweise adhäsiven Kittsubstanz (115) statt.

Die Figur 20 zeigt einen mittels Gestellvorrichtung und Halteelementen (140) fixierten Funktionskopf mit einer, durch das Auflagekissen (116) oder durch das zu einem geschlossenen Ring geformte elastische Hohlkörperkissen (117) mit Druckanschluß (nicht gezeichnet) und Trennfolie (118) mit Befestigung deren (144) am Gestell gebildeten Kammer (119).

Die Figuren 21 bis 23 zeigen eine Trennvorrichtung in vivo mit Abdichtung im Zahnfleischsulkus (120) durch einen in diesen applizierten, über seine Länge einseitig mit Löchern oder Schlitzen (121) versehenen, von einem elastischen, die Kittsubstanz (123) aufnehmenden Material (124) ummantelten, leicht biegsamen Schlauch (125) mit Druckanschluß an einen die Kittsubstanz enthaltenden Behälter (nicht gezeichnet) [Fig 21/22] und mit Verbindung zur Trennfolie, oder ein faden-/ringförmigen Träger (149) [Fig.23] mit der Kittsubstanz (123), der mit der Trennfolie (126) fest verbunden ist.

Zur weiteren Fixierung der Trennfolie kann diese durch ein weiteres chemoplastisches adhäsives Material (127) [Fig. 21/22] an den Nachbarzähnen befestigt werden. Die Trennfolie kann dabei auch eine besonders einfache und platzsparende Anbringung des kathodischen Anschlusses (128) ermöglichen, indem die mit dem adhäsiven Material an der Zahnoberfläche befestigte Trennfolie (126) nach der Leitmetallauftragung und nach Applikation des Kathodenanschlusses an der Klebegrenzlinie (129) diese bedeckend auf die metallische Zahnoberfläche (131) geklebt wird. Dabei bleibt jedoch die Objektoberfläche unbedeckt.

Die Figur 22 kann als Querschnittdarstellung der Figur 21 durch den Objektzahn angesehen werden. Zur weiteren Fixierung, insbesondere auf der lingualen Seite, ist die Trennfolie mit den Verbindungsstangen (132) einer an den Nachbarzähnen fixierten Gestellvorrichtung (nicht gezeichnet) befestigt. Im Arbeitsfeld (133) sind drei Funktionsenden -(134) rotierende Anode zylindrisch, (135) schwingende Anode, (136) Sprühgerät - andeutungsweise dargestellt und ein Absaugkopf (137) einer externen Absaugvorrichtung mit Wattebelegung (138) und Absauganschlüssen (139) skizziert.

Figur 23 zeigt eine Gestellvorrichtung mit Befestigungselementen (140) an den Nachbarzähnen, Verschlußdeckel (141) mit Zu-(142) und Abführungsleitungen (143) und Befestigungsmittel (144) für die Trennfolie. Die Zu- und Abführungsleitungen enden dabei in einer individuell für den Objektzahn hergestellten Negativ-Form (148) zur besseren und gleichmäßigen Strömumngsbeeinflussung, dieser dient bei der Hochgeschwindigkeitsgalvanisierung auch als Anode.

Figur 24 a zeigt zwei Flüssigkeitsbehälter, jeweils in der Gestalt einer Zweikammerspritze mit vergleichsweise kleinem Lumen, Anschluß an die Zuführungsleitung (145) über einen Dreiwegehahn (146) und einen flüssigkeitsdurchlässigen, mittels eines weiteren Dreiwegehahnes (147) verschließbaren, an der Bewegungsvorrichtung (nicht gezeichnet) angeschlossenen Kolben mit Anschluß an den Vorratsbehälter (VA) und (VB). Die Bewegungsrichtung ist durch große Pfeile angedeutet.

Die Schalterstellung der beiden Dreiwegehähne bewirkt: In der Zuführungsvorrichtung A ihre Abschaltung bei Betrieb der Bewegungsvorrichtung mit Folge einer Flüssigkeitsbewegung ausschließlich im Kolbenlumen (siehe Pfeile), in der Zuführungsvorrichtung B eine Flüssigkeitsbewegung aus dem Kammerlumen in die Zuführungsleitung und gleichzeitig einen Nachfluß der Flüssigkeit aus dem Vorratsbehälter in die gegenüberliegende Kammer (siehe Pfeile).

In Figur 24 b ist die Schalterkombination für die unveränderte .Flüssigkeitszufuhr nach Änderung der Bewegungsrichtung in der Zuführungsvorrichtung B dargestellt.

## Ansprüche

I.1. Gerät zum Aufbringen eines metallischen Stoffes auf Zähnen und auf den Zähnen befindlichen körperfremden Materialien, gekennzeichnet durch eine an einen Behälter für eine Metall enthaltende Flüssigkeit angeschlossene Auftragvorrichtung und eine weitere, mit dieser zu einer Einheit zusammengebauten, Auftragvorrichtung für das Mittel zum Abscheiden des Metalls, vorzugsweise ein Reduktionsmittel, und Vorrichtungen für die gleichzeitige oder in kleinen Zeitintervallen alternierend erfolgende Zuführung zu den Auftragvorrichtungen, wobei die Auftragvorrichtungen mit ihren Ausgängen auf einen gemeinsamen Ort oder zwei dicht nebeneinander liegenden Orten gerichtet sind.

I.2. Gerät nach Anspruch I.1, dadurch gekennzeichnet, daß die an den Behälter für eine Metall enthaltende Flüssigkeit angeschlossene Auftragvorrichtung aus einem an den Behälter mit einer Vorrichtung zur Druckbeaufschlagung angeschlossenen, am anderen Ende offenen Rohr und/oder einem Spritzgerät und/oder einem mit Ultraschall und/oder hydraulisch und/oder pneumatisch betriebenen Sprüh- und/oder Zerstäubungsgerät mit Aerosolbildung im Bereich des Endes der Auftragvorrichtung und/oder vor diesem, vorzugsweise in einer zwischengeschalteten Kammer besteht und die Auftragvorrichtung für das Mittel zum Abscheiden des Metalls aus einem an einen Behälter für eine Flüssigkeit und/oder ein Gas und/oder ein Festkörper-Gas- und/oder Festkörper-Flüssigkeit-Gemisch mit einer Vorrichtung zur Druckbeaufschlagung angeschlossenen, am anderen Ende offenen Rohr und/oder einem Spritzgerät und/oder

einem mit Ultraschall und/oder hydraulisch und/oder pneumatisch betriebenen Sprüh- und/oder Zerstäubungsgerät mit Aerosolbildung im Bereich des Endes der Auftragvorrichtung und/oder vor diesem, vorzugsweise in einer zwischengeschalteten Kammer besteht, und/oder

einer Lichtquelle, einem Laser, und/oder

einer Wärmequelle mit gerichteter Wärmeabgabe besteht und/oder

eine Wärmequelle und/oder Lichtquelle aufweist und/oder daß

der Behälter der Auftragvorrichtung an eine oder zwei weitere Behälter mit den Komponenten der zuzuführenden Substanz und/oder an Vorrichtungen zur Druckbeaufschlagung oder Vermischungsbewegung angeschlossen ist,

die Auftragvorrichtung(en) an eine Bewegungsvorrichtung angeschlossen sind und/oder daß

die Auftragvorrichtungen eine Vorrichtung zur Verstellbarkeit ihrer Zuführungen als Parameter der Zuführungsprodukte aufweisen und/oder daß

die Auftragvorrichtungen für die Metall und Abscheidungsmittel enthaltende Flüssigkeit in einer Auftragvorrichtung mit getrennten Zuleitungen untergebracht sind und/oder daß

die Auftreff-Fläche der Zuführungen auf dem Objekt punkt-bzw. kreisförmig oder strichförmig bzw. oval ist.

I.3. Gerät nach Anspruch I.1,
gekennzeichnet durch

eine Vorrichtung zum Abführen der aufgetragenen Substanzen, Stoffe und Reaktionsprodukte nach der Abscheidungsreaktion und/oder mit einer Verbindung zu einem Flüssigkeit speichernden Material und/oder

eine Vorrichtung zur Fixierung und/oder Änderung der relativen Position des Funktionsendes zum Handstück und/oder

eine Vorrichtung zum Verstellen und Fixieren der relativen Position der Auftragvorrichtungen zueinander und/oder

einen Distanzhalter zur Festlegung der minimalen Entfernung zwischen Auftragvorrichtungen und Objektoberfläche,

eine Heiz- und/oder Kühlvorrichtung zur Temperatureinstellung der zugeführten und/oder bevorrateten Stoffe oder Substanzen und/oder Zerstäubungsgase und/oder

eine oder mindestens zwei Lichtleiterenden und/oder Lichtquellen und/oder optische (bzw. elektromagnetische) Linsen und/oder Filter an den Auftragvorrichtungen, deren Strahlen gleicher oder verschiedener Wellenlängenzusammensetzung sich kreuzen, wobei der Kreuzungspunktpunkt vorzugsweise in der Ebene des Zusammentreffens der Auftragvorrichtungen senkrecht zur Zuführungsrichtung liegt, und/oder

ein Sender-Empfängersystem für optische (elektromagnetische) und/oder akustische Strahlen (Wellen) und/oder

Detektoren für Temperatur und/oder Druck und/oder elektrischen Widerstand und/oder

einen Steuer-Regelkreis mit Anschluß an die Sender-Empfängersysteme und/oder an die Detektoren und/oder an die Verstellvorrichtungen und/oder an die Vorrichtungen zur Druckbeaufschlagung und/oder an die Heiz-Kühlvorrichtung und/oder an die Vorrichtungen zur Verstellbarkeit der Zuführungen der Auftragvorrichtungen, als Parameter der Zuführungsprodukte und/oder an ein Informationsübertragungssystem.

II.1. Gerät zum Aufbringen eines metallischen Stoffes auf Zähnen und auf den Zähnen befindlichen körperfremden Materialien,
dadurch gekennzeichnet, daß es

eine Stromquelle aufweist, deren Kathode für die Anbringung an der zu behandelnden zumindest mit einer Leitmetallschicht versehenen Oberfläche eingerichtet ist,

die Anode mit einem flüssigkeitsdurchlässigen und flüssigkeitsspeichernden Material zumindest teilweise bedeckt ist welches selbst nicht leitfähig ist, und daß

Anode und/oder deren bedeckendes Material von einer Bewegungsvorrichtung angetrieben ist und/oder daß

die Anode und ihr bedeckendes Material länglich flächig gestaltet ist und eine Vorrichtung für eine manuelle Bewewgung aufweist.

II.2. Gerät nach Anspruch II.1 und/oder III.1,
dadurch gekennzeichnet, daß
die Bewegungsvorrichtung

ein rotierender und /oder schwingender Antrieb für eine hin und hergehende Bewegung ist, und/oder daß

die Anode und/oder ihre Ummantelung
eine rotationssymmetrische Form und/oder über ihre Länge unterschiedliche Radien aufweist und/oder

der Form des zur Präparation des zu beschichtenden Teilflächenabschnittes der Kavitätenwandung verwendeten rotierenden Schleifinstrumentes entsprechend mit abweichender oder gleicher Größe gestaltet ist und/oder

mit abweichender Form, vorzugsweise im Bereich des entsprechenden Schleifinstrumentenendes und/oder darüber hinaus, gestaltet ist und/oder

der Form und Größe dem Lumen der zu beschichtenden Wurzelkanalwandung und/oder des zur Wurzelkanalaufbereitung verwendeten (normierten) Instrumentes entsprechend, mit abweichender oder gleicher Größe und/oder Form, insbesondere in seinen über die Längsachse angeordneten Teilabschnitten und/oder in Abhängigkeit der eingestellten Instrumentenlänge in Relation zur Wurzelkanallänge, vorzugsweise mit verkleinerten Querschnitt in dem zum Instrumentenhalter gerichteten (proximalen) Anteil und gleicher oder geringfügig veränderter Größe an der Instrumentenspitze (distal) gestaltet ist und/oder

flächenförmig gestaltet ist und/oder

nur einseitig von dem flüssigkeitsspeichernden Material bedeckt ist und /oder

in ihrer Form der oder den zu beschichtenden Teiloberflächen in einer Orientierung entsprechend gestaltet ist, so daß eine eindimensionale kongruente Anlagerung möglich ist

an eine Vorrichtung mit einem Behälter, vorzugsweise mit einer Vorrichtung zur Überdruckerzeugung auf die Flüssigkeit, für die Zuführung der das abzuscheidene Metall enthaltenden Flüssigkeit und/oder

an eine Vorrichtung mit einem Behälter, vorzugsweise mit einer Vorrichtung zur Unterdruckerzeugung auf die Flüssigkeit, der mit dem Behälter der Zuführungsvorrichtung in Flüssigkeitsverbindung gebracht werden kann, für die Abführung der Flüssigkeit angeschlossen ist und/oder

interne und/oder externe Leitungen für Flüssigkeitstransport und/oder elektrischen Leitungen aufweist und/oder

Elektroden mit einem Anschluß an eine Vorrichtung zur Widerstandsaufnahme zur Bestimmung des Widerstands des Flüssigkeit speichernden Materials und/oder des in der Anode transportierten oder befindlichen Elektrolyten, versehen ist und/oder

aus einem in dem Flüssigkeit aufnehmenden Material eingelegten und/oder eingeflochtenen und/oder gewickelten Metalldraht, -band, -netz oder -gitter besteht, dessen äußere für den Kontakt mit der Kathode vorgeshene Oberfläche ausschließlich vom Ummantelungsmaterial in vergleichsweise kleiner Stärke aufgenommen wird und/oder dessen innere, zur Verbindung mit dem Anodenhalter vorgesehene, Oberfläche einen elektrischen Anschluß aufweist und/oder als ein aufzusteckender Körper gestaltet ist und/oder dessen innere Fläche durch ein festes Rahmengerüst gebildet ist und/oder zur Aufnahme des nicht rotationssymmetrischen Anodenhalters vorgesehen ist und/oder eine feste Verbindung mit dem Ummantelungsmaterial eingeht und/oder daß

die Anode

an eine Vorrichtung zur Widerstandsmessung zur Bestimmung der Widerstands zwischen Anode und Kathode angeschlossen ist und/oder

mehr als eine Anodenteilfläche mit je einem Anschluß an eine Spannungsquelle mit zusammengefaßter Kathode und/oder einen Anschluß an je eine Vorrichtung für die Widerstandsbestimmungen aufweist und /oder

aus einem teleskopartig zusammengefügten Kanülensystem aufgebaut ist und/oder die einzelnen Kanülen und/oder ihre Anteile elektrisch leitend und/oder nichtleitend miteinander verschweißt und/oder verlötet und/oder verklebt und/oder per Friktion miteinander verbunden sind und/oder ihrer Länge nach geteilt sind und/oder

aus einem System über- und/nebeneinander angeordneter metallischer Folien, die mit, den elektrischen Strom leitenden und/oder nichtleitenden Zwischenschichten mit einander verbunden sind und/oder

bis auf ihre Spitze vollständig elektrisch isoliert ist und/oder an eine Vorrichtung zur Messung des Widerstands zwischen ihr und der Objektoberfläche angeschlossen ist und/oder daß

das Flüssigkeit speichernde Material als ein auf die Anode aufzusteckender Körper gestaltet ist und/oder

an der Anode geklebt und/oder gewickelt und/oder gespannt und/oder chemoplastisch und/oder über Retention an der Anodenfläche befestigt wird und/oder

in Bereichen spitzer, scharfer Ecken und Kanten verstärkt ist und/oder daß

die Spannungsquelle(n) der Abscheidungselektroden an eine Steuervorrichtung zur Änderung und/oder Einstellung der Spannung im Sinne der Regulation (Steuer- Regelkreis) und/oder alternierenden Steuerung (Impulseplating/ Sinusfunktion für schwingenden Antrieb) und/oder Umpolung (anodische Oxidation; Passivierung) aufweisen und/oder daß

es eine Vorrichtung zum Heizen und/oder Kühlen der Flüssigkeiten und/oder Temperaturfühler und/oder Druckrezeptoren zur intra- und/oder extraluminalen Druckmessung aufweist und/oder daß

es eine Steuer-Regelvorrichtung und/oder ein akustisches und/oder visuelles Informationsübertragungssystem mit Anschluß an die Bewegungsantriebe und/oder an den Vorrichtungen der Wiederstandsaufnahme für die Elektrolyte in den luminalen Bereichen und/oder Ummantelung und/oder zwischen Anode und Kathode und/oder an den Druck- und/oder Temperaturfühlern und /oder an den Vorrichtungen zur Zuführung und/oder Abführung und/oder Temperatursteuerung und/oder an die Steuervorrichtung für die Spannungsquellen der Abscheidungselektroden aufweist und/oder daß

der Kathodenanschluß für die Zahnoberfläche eine adhaesive (klebrige) Fläche und/oder eine Vorrichtung zur Unterdruckerzeugung und/oder

einen mit chemoplastischem adhaesivem Material gefüllten Napf aufweist und/oder in eine Trennvorrichtung untergebracht ist.

III.1. Gerät zum Aufbringen eines metallischen Stoffes auf Zähnen und auf den Zähnen befindlichen körperfremden Materialien,
dadurch gekennzeichnet, daß es
einen Behälter für eine Metall enthaltende Flüssigkeit,
eine Vorrichtung zur Druckbeaufschlagung auf die Flüssigkeit,
eine Vorrichtung zum Auftragen der Flüssigkeit mit einer flüssigkeitsführenden Anschlußleitung an den Behälter und
eine Vorrichtung zum Abführen der Flüssigkeit mit einer flüssigkeitsführenden Anschlußleitung an einen Behälter aufweist und daß
die Abführung und Zuführung gleichzeitig oder in vergleichsweise kleinen Zeitabständen alternierend stattfindet.

III.2. Gerät nach Anspruch III.1,
dadurch gekennzeichnet, daß
die Auftragvorrichtung
ein an den Behälter angeschlossenes Austrittsrohr enthält und/oder
an einem Behälter für eine Reduktionsmittel und Metall enthaltende Flüssigkeit angeschlossen ist und/oder
an einem weiteren Behälter für eine Reduktionsmittel enthaltende Flüssigkeit angeschlossen ist, dessen flüssigkeitsführendes Rohr in das Austrittsrohr für die Metall enthaltende Flüssigkeit oder in einem eigenen, zweiten Austrittsrohr der Auftragvorrichtung endet und/oder
mit dem Anschluß an den Behälter für die Metall und/oder das Mittel zum Abscheiden enthaltende Flüssigkeit ein Spritz- und/oder auf unterschiedliche Weise betriebenes Sprühgerät darstellt und/oder daß
es eine Abscheidevorrichtung aufweist, die
eine an einem Behälter für das Mittel zum Abscheiden enthaltende Lösung angeschlossene Auftragvorrichtung und/oder
eine Stromquelle, deren Kathode für die Anbringung an der zu behandelnden, zumindest mit einer Leitmetallschicht versehenen Oberfläche eingerichtet ist, und deren Anode mit einer zumindest Metall enthaltenden Flüssigkeit in Verbindung steht und/oder die aus mehreren Anodenteilflächen zusammengesetzt ist mit Anschluß an je eine Stromquelle mit zusammengefaßter Kathode und/oder mit Anschluß an den Steuer-Regelkreis und/oder von einem Flüssigkeit speichernden und durchlässigen elektrisch nicht leitfähigen Material bedeckt ist und/oder
eine Licht- (Laser-) und/oder Wärmequelle aufweist oder darstellt und/oder daß
die Absaugvorrichtung
eine Vorrichtung zur Unterdruckerzeugung und/oder ihr Behälter mit dem der Auftragvorrichtung eine Verbindung aufweist und/oder an ihrer(n) Absaugöffnung(en) mit einem flüssigkeitsspeichernden und -durchlässigen Material in Verbindung steht und/oder ihre Öffnung trichterförmig und in Aufsicht der Form des Behandlungsausschnittes entsprechend, vorzugsweise oval oder eiförmig, gestaltet ist und/oder daß
Auftrag-, Absaug- und Abscheidevorrichtung, falls vorhanden, in einem gemeinsamen Funktionskopf untergebracht sind und/oder eine Bewegungsvorrichtung aufweisen

III.3. Gerät nach einem der Ansprüche III.1, I.1 oder II.1,
dadurch gekennzeichnet, daß es
einen Funktionskopf aufweist
mit einem Auflagekissen,
dessen Rand zumindest teilweise gegenüber seinem zentralen Anteil erhöht ist, in dem Auftrag- und/oder Abscheidevorrichtung und/oder Absaugvorrichtung enden, und/oder
dessen unterhalb des Auflageniveaus liegende, vom Auflagekissenrand begrenzte Kammer durch ein zwischen den Enden der Absaug- und Auftragvorrichtung angeordneten Anteil mit oder geringfügig unterhalb der Höhe des Auflagekissenrandes die zentrale Kammer in eine Kammer mit Anschluß an die Auftragvorrichtung und in eine weitere Kammer mit Anschluß an die Absaugvorrichtung unterteilt und/oder die Abscheidevorrichtung im zwischengeschalteten Anteil und/oder der Kammer mit der Auftragvorrichtung untergebracht ist und/oder diesen bildet und/oder zumindest teilweise seine Oberfläche einnimmt und/oder
der zwei gegenüber liegende Auflageflächen aufweist und/oder dessen zentrale Kammer von zwei Objektoberflächenabschnitten begrenzt wird und/oder
das aus einem elastisch verformbaren Material und/oder einem Flüssigkeit speichernden Material und/oder einen elastisch verformbaren, gas- und/oder flüssigkeitsgefüllten Hohlkörper und/oder mit einem Anschluß an eine Vorrichtung zur Druckbeaufschlagung aufweist und/oder
der an seiner Seiten- und/oder Rückfläche einen elastisch verformbaren, gas- und/oder flüssigkeitsgefüllten Hohlkörper mit oder ohne Anschluß an eine Vorrichtung zur Druckbeaufschlagung aufweist und/oder
der flächen- und/oder scheiben- und/oder band- und/oder ringförmig gestaltet ist und/oder
der mit einem flüssigkeitsdurchlässigen und -speichernden Material bedeckt oder gefüllt ist, in dem zumindest die Auftragvorrichtung und falls vorhanden die Anode der Abscheidevorrichtung und/oder mit paralleler Anordnung zur Auflagefläche endet.

III.4. Gerät nach einem der Ansprüche III.1, I.1 und II.1,
dadurch gekennzeichnet, daß
Auftrag- und/oder Abscheide- und/oder Absaugvorrichtung
in Form und Größe des Lumens der zu beschichtenden Wurzelkanalwandung und/oder des zur Wurzelkanalaufbereitung verwendeten (normierten) Instrumentes entsprechend, mit abweichender oder gleicher Größe und/oder Form, insbesondere in den über die Längsachse angeordneten Teilabschnitten und/oder in Abhängigkeit der eingestellten Instrumentenlänge in Relation zur Wurzelkanallänge abweichend gestaltet sind und/oder

in einer Vorrichtung zum Festhalten und/oder veränderlichen Einstellen ihrer freien Länge, vorzugsweise über eine an der Vorrichtung enthaltenen Referenzauflage zur vertikalen Fixierung des Funktionsendes am Zahn untergebracht ist, das für die bidigitale Handhabung und Bewegung mit einem Griff und/oder für den Anschluß an eine Bewegungsvorrichtung vorgesehen ist und/oder eine kontinuierliche und/oder schrittweise Arbeitslängenverstellung ermöglicht und/oder mit einem elastisch verformbaren Kissen und/oder Hohlkörper mit Anschluß an eine Vorrichtung zur Druckbeaufschlagung mit oder ohne, durch seine Wandstärke festgelegter spezifisch lokalisierter Expansion ausgestattet ist und/oder

an eine Bewegungsvorrichtung angeschlossen sind und/oder

mit ihren Zuführungsleitungen im Bereich der Spitze des Funktionsendes oder -kopfes enden und/oder die Austrittsrichtung der Zuführungen aus ihren Öffnungen parallel und/oder senkrecht und/oder in einem spitzen Winkel zur Längsachse eingestellt sind und/oder ihre Austrittsöffnungen einen vergleichsweise großen, vorzugsweise runden und/oder einen vergleichsweise kleinen mit vorzugsweise ovalem, spalt- oder schlitzförmigen Querschnitt und/oder mehr als eine Austrittsöffnung je Zuführungsvorrichtung aufweisen mit Anordnung in gleicher und/oder unterschiedlicher Höhe und/oder nebeneinander angeordneten Öffnungen der Auftrag- und Abscheidevorrichtungen und/oder aus einem flexiblen und torsionsstabilen Material bestehen und/oder

mit ihrer Abführungsleitung in einer Mindestentfernung von den Austrittsöffnugen der Zuleitungen, vorzugsweise im Bereich des Wurzelkanaleingangs enden und/oder den Wurzelkanal an dieser Stelle durch Anlagerung und/oder mittels eines elastischen Hohlkörpers abdichten und/oder

mit ihren gesamten Zuführungsraten gleich oder kleiner als die Abführungsrate sind und/oder die Größe und Form der Funktionsenden derart gestaltet sind, daß die Querschnittsfläche zwischen ihnen und der Wurzelkanalwandung zum Absaugrohr zunnimmt und/oder daß

sie eine Vorrichtung zur substanzdichten Trennung des in die Mundhöhle gerichteten Anteils eines Zahnes von seinem wurzelspitzenwärts gelegenen Anteil und den umliegenden anatomischen Strukturen und/oder einen faden- und/oder band-und/oder ringförmigen Träger und/oder einen Hohlkörper, zur Applikation in den Zahnfleischsulkus, mit senkrecht zu seiner Achse angeordneten Austrittsöffnungen und/oder zur Aufnahme eines chemoplastischen Materials und/oder mit Anschluß an einen Behälter für die Komponenten des Chemoplasten und/oder mit einer Vorrichtung zur Druckbeaufschlagung und/oder mit Befestigung an eine Folie und/oder Trennwand und/oder einer Gestellvorrichtung zur Fixierung der freien Anteile und/oder mit Zahnklemm- und Zahnhalteelementen aufweisen und/oder mit ihren Zuführungsleitungen und Abführungsleitungen an strömumgstechnisch gegenüberliegenden Stellen des Objektzahnes angeordnet sind und/oder die Anode und/oder der Funktionskopf eine konfektionierte und/oder individuell für den Zahn hergestellte Negativform mit Aussparung des Durchflußlumens zwischen ihr und Objektoberfläche darstellt und/oder für ihre Befestigung und/oder mit dem kathodischen Anschluß an die Objektoberfläche als Distanzhalter vorgesehen ist.

III.5. Gerät nach einem der Ansprüche I.1, II.1, und III.1,
dadurch gekennzeichnet, daß
der Behälter einer Auftragvorrichtung und/oder Abscheidevorrichtung aus einem vorzugsweise zylinderförmigen Gefäß besteht mit einem dieses in zwei Kammern unterteilenden verschieblichen Kolben mit einer verschließbaren vorzugsweise großlumigen Verbindungsleitung beider Kammern und/oder an ein oder mehrere Behälter oder Patronen oder Mischkapseln angeschlossen ist, die die zu vermischenden Komponenten der Flüssigkeit der Vorrichtung enthalten.

III.6. Gerät nach einem der Ansprüche I.1, II.1, und III.1,
dadurch gekennzeichnet, daß
die Vorrichtung zur Druckerzeugung mechanisch über eine rotierbare Gewindestange mit Anschluß an einen Motor beider Drehrichtungen betrieben ist und daß durch unterschiedliche Drehrichtung ein Unterdruck oder Überdruck erzeugt wird und daß die Flüssigkeit enthaltenden und führenden Behälter und Leitungen für Druckbeanspruchung ausgelegt sind und/oder an ein Leitungsschaltungssystem angeschlossen sind mit Anschluß an einen Druckgasbehälter und an weitere Behälter für Substanzen enthaltende Flüssigkeiten für vor- oder zwischenbehandelnde Maßnahmen (Spülung, Ätzung, Bekeimung, Aktivierung).

III.7. Gerät nach einem der Ansprüche I.1, II.1, und III.1,
dadurch gekennzeichnet, daß es ein oder mehrere Geräte oder Vorrichtungen aufweist, deren Flüssigkeitsbehälter, Vorrichtung zur Druckbeaufschlagung, Absaugpumpe, Auffangbehälter, Stromquelle, elektronische Schaltungen, Steuer-Regelvorrichtungen und dergleichen in einer Tisch-oder Wagenbox oder in einer zahnärztlichen Behandlungseinheit untergebracht sind, deren Funktionskopf oder -ende an einem Handstück mit Behandlungsaufsatz angeordnet sind und deren Verbindungsleitungen in einem flexiblen Zuleitungsschlauch untergebracht sind, und ein oder mehrere Elemente eines Gerätes oder einer Vorrichtung Bestandteil mehrerer Vorrichtungen sind.

EP 0 392 424 A1

Fig. 1

6

2

4 7

5

3

1

Fig. 2

5

7

4

6

11

11

14

8

8

8

8

10

12

15

9

13

15

16

17

15

α

β

Fig. 3

Fig. 4

Fig. 5

Fig. 6

Fig. 7

a

b    21  19

c    36  18  20  36  21  19  36

Fig. 8

a    e    b    c

24    36    37

d

b    20  18  19  21

c

d    20  18  19  21

e    23

Fig. 9

a    e    b    c

23

d    36  18  19

b    20  21

c

d    20  18  37  21

e    24

Fig. 10

Fig. 11

Fig. 12

Fig. 13

## Fig. 14

## Fig. 15

Fig. 16

a

+  −

9

b        c        d

27   30   99   103  98   101   100   102

b

c

99   27

d

100

103

e

27

99

98

101

Fig. 17

99

98

101

27

19

9

a

b

**Fig. 18**

**Fig. 19**

**Fig. 20**

**Fig. 21**

**Fig. 22**

Fig. 23

144　141　　142　143

OL

UL

140　126　149　148　123

EP 0 392 424 A1

Fig. 24

**Europäisches Patentamt**

# EUROPÄISCHER RECHERCHENBERICHT

| EINSCHLÄGIGE DOKUMENTE | | | EP 90106770.2 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl⁵) |
| D,A | US - A - 3 995 371 (THOMAS J. O'KEEFE) * Zusammenfassung * -- | I1,I | A 61 C 5/00 |
| D,A | FR - A - 2 287 208 (THE CURATORS OF THE UNIVERSITY OF MISSOURI) * Patentanspruch 1 * ---- | I1,I | |

|  | RECHERCHIERTE SACHGEBIETE (Int Cl⁵) |
|---|---|
|  | A 61 C |

Der vorliegende Recherchenbericht wurde für alle Patentansprüche erstellt.

| Recherchenort WIEN | Abschlußdatum der Recherche 06-07-1990 | Prüfer HEIN |
|---|---|---|